# EUROPEAN PATENT APPLICATION

(11) **EP 4 541 801 A1**
(43) Date of publication of application: **23.04.2025**
(21) Application number: 23826213.3
(22) Date of filing: 13.06.2023
(51) Int. Cl.: C07F 13/00, A61K 49/06

(54) **MARKED FATTY ACID DERIVATIVE, PRECURSOR COMPOUND THEREOF, AND USE THEREOF IN CONTRAST AGENT**

(30) Priority: 20.06.2022 CN 202210707798
(71) Applicant: Beijing Normal University, Beijing 100875 (CN)
(72) Inventor: ZHANG, Huabei, Beijing 100875 (CN); FANG, Yu, Beijing 100875 (CN)
(74) Representative: Lippert Stachow Patentanwälte Rechtsanwälte Partnerschaft mbB
(86) International application number: PCT/CN2023/099946
(87) International publication number: WO 2023/246564

(57) **Abstract**

The present invention relates to a marked fatty acid derivative, a precursor compound thereof, and use thereof in a contrast agent. The derivative is represented by formula I: wherein M is ^{99m}Tc or Re; R, R¹, R², R³, R⁴ and R⁵, and A₁-A₁₂ are independently H, an aliphatic chain or an alicyclic; J, X, and Y are independently absent, or -O-, -S- or (II), and J, X, and Y are not simultaneously absent; R⁶-R¹⁴ are independently H or an aliphatic chain, and R¹³ and R¹⁴ are not H simultaneously; z is 1-6, a is 0-5, b is 0-5, and c is 0-3; d is 0-27, e is 0-27, f is 0-28, g is 0-7, and at least two of d, e, f and g are not 0. The present invention provides a completely novel structure type that can be used for preparing a novel myocardial metabolism contrast agent, possessing important clinical application prospects.

## Description

### FIELD

The invention relates to the field of medicine and chemical technology, and in particular to a class of labeled isocyanate-containing fatty acid derivatives, precursor compounds thereof and applications thereof in inventions.

### BACKGROUND

Fatty acids are the simplest type of lipid, and they are the components of many more complex lipids. Fatty acids can be oxidized and decomposed into CO₂ and H₂O when there is sufficient oxygen supply, releasing a large amount of energy. Therefore, fatty acids are one of the main sources of energy for the body, especially the main source of energy for myocardial contraction.

The role of fatty acids in the myocardium The process of muscle contraction requires high energy, so continuous and efficient ATP is necessary to maintain contractile function, basal metabolism and ion exchange balance. In normal human myocardium, the oxidation of fatty acids can provide 60%-80% of the energy required by the myocardium. In addition, the myocardium can also obtain the energy required for physiological activities by oxidizing energy substances such as glucose, pyruvate, amino acids and lactic acid. When the body is in a state of low blood sugar concentration or fasting, the heart has a strong ability to absorb fatty acids, and at this time, almost all of the oxygen consumption of the myocardium is provided for the oxidation of fatty acids.

When the myocardium is adequately supplied with oxygen, fatty acids can efficiently provide energy to the myocardium through *β*-oxidation; when the myocardium is ischemic or has low oxygen supply, the *β*-oxidation of fatty acids is inhibited and converted into sugar metabolism to provide energy for myocardial metabolism, and the utilization rate of fatty acids by the myocardium decreases.

The metabolic pathway of myocardial fatty acid imaging agents is basically similar to that of free fatty acids. The oxidation of fatty acids is divided into three steps. The first step is the activation of fatty acids in the cell fluid. Fatty acids are catalyzed by acyl-CoA synthetase to produce acyl-CoA. The second step is the transport of acyl-CoA into the mitochondria. The third step is the β-oxidation of acyl-CoA. After a β-oxidation, long-chain fatty acids lose acetyl-CoA with two carbon atoms starting from the β carbon atom to generate acyl-CoA with two carbon atoms less, releasing a large amount of energy in the process.

The β-oxidation of acyl-CoA in the myocardium is divided into four steps, namely, removal of one H at the α and β positions, addition of water, dehydrogenation to generate β-acyl-CoA, and sulfhydrylation. Each step corresponds to a different enzyme: acyl-CoA dehydrogenase, enoyl-CoA hydratase, 3-OH acyl-CoA dehydrogenase, and β-ketoacyl-CoA thiolase.

At present, the myocardial metabolic imaging agents used in clinical practice mainly include: [¹¹C]-palmitate and [¹⁸F]-FDG for PET imaging, and [¹²³I]-IPPA and [¹²³I]BMIPP for SPECT imaging. Among them, the half-life of ¹¹C is only 110 min, which has high requirements for the speed of labeling and imaging. ¹⁸F-FDG is relatively expensive, and radioactive iodine-labeled drugs are easily deiodinated in the body, and radioactive iodine-labeled drugs must be purchased from specialized companies. These different drugs have their own shortcomings that cannot be overcome. ^{99m}Tc has excellent radionuclide properties. Its half-life is 6 h, which is convenient for drug labeling, transportation and use. It is also inexpensive and easy to obtain. Another extremely important advantage is that ^{99m}Tc has different valences (+1 to +7) and can form a variety of different coordination structures, with coordination numbers of 5, 6, and 7. Among them, there is a labeling method that has been realized in a drug box, which is convenient for clinical application. However, there is still no ^{99m}Tc-labeled myocardial metabolic imaging agent that has been successfully used in clinical practice.

Then in 2008, Byung Chul Lee *et al.* introduced a carbonyl group based on the structure of the compound [^{99m}Tc]CpTT-PA, designed and synthesized the compound ^{99m}Tc-CpTT-16-oxo-HAD, with the purpose of increasing hydrophilicity and reducing liver uptake. The compound is metabolized in the myocardium by β-oxidation, and finally metabolized into the compound ^{99m}Tc-CpTT-4-oxo-butyric acid, which has a higher myocardial uptake value than the compound [^{99m}Tc]CpTT-PA, but the liver background, lung background and kidney background are high, and the ideal target to non-target ratio is not obtained.

In 2012, Zeng Huahui *et al.* introduced an amide bond into the long-chain structure of compound ^{99m}Tc-CpTT-16-oxo-HAD and synthesized compound ^{99m}Tc-CpTT-6-oxo-HAUA. The compound had a reduced level in the liver, but the myocardial uptake value was relatively low, at only 4.37% ID/g at 1 min.

In order to solve the above technical problems, the applicant/inventor also studied isocyanate-containing (labeled) fatty compounds. Although the overall effect was significantly improved, there were still shortcomings such as high liver background and lung background, and poor myocardial imaging effect, which generally affected further drug development. Therefore, in order to better achieve real clinical application, the inventor's research group has been conducting research in different directions and structures for nearly three years. Through hard work, it was found that the structure in this application has excellent overall effect.

### Summary

In view of this, the purpose of the present invention is to provide a labeled fatty acid derivative, a precursor compound thereof and the use thereof in the invention, which solves the deficiencies in the prior art. The purpose of the present invention is achieved through the following technical solutions:

One of the inventive aspects of the present invention is to provide a labeled fatty acid derivative, the general formula of which is shown in Formula I:

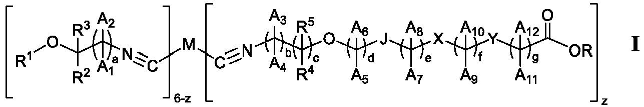

Wherein, M is ^{99m}Tc or Re; R, R¹, R², R³, R⁴ and R⁵ are independently H, aliphatic chain or alicyclic, and these six groups are completely identical, completely different or partially identical groups; A1~A12 (i.e., A1, A2, A3, A4, A5, A6, A7, A8, A9, A10, A11, A12) are independently H, aliphatic chain or alicyclic; J, X, Y are independently none, -O- (oxygen atom), -S- (sulfur atom), 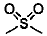 (sulfone), 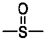 (sulfoxide), 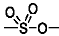 -(sulfonic acid group), 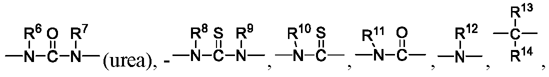 and J, X, Y cannot be none at the same time, R⁶~R¹⁴ are independently H or aliphatic chain, and R¹³~R¹⁴ cannot be H at the same time; z is an integer from 1 to 6, a is an integer from 0 to 5, b is an integer from 0 to 5, c is 0 to 3, and preferably b and c are not 0 at the same time. d is an integer from 0 to 27, e is an integer from 0 to 27, f is an integer from 0 to 28, and g is an integer from 0 to 7, and in a specific compound, at least two of d, e, f and g are not 0, that is, preferably, in the structure on the right side of M, the adjacent two ether oxygen groups O, J, X, Y and COOR are not directly connected, but are connected through at least one C atom, such as: if J and X are absent, there is no direct connection between O and Y and between Y and COOR.

Furthermore, among J, X, and Y, at least one is an etheroxy group or a group containing a S atom, or the group 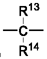 containing a S atom includes at least one of -S-,

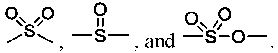

Furthermore, J, X, and Y are independently none, -O-, -S-, 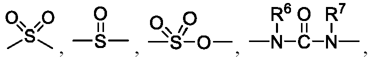 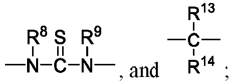 preferably, J is none, 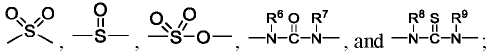 Xis none, 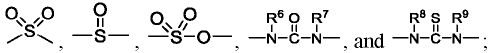 Y is -O-, -S-, 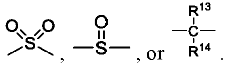 More preferably, at least one of J, X, and Y is a group containing S atoms or 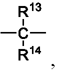 and the group containing S atoms includes -S-,

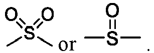

More preferably, in the general formula I, J, X, and Y are more preferably any one of the following combinations: (1) J and X are both none, and Y is -S-, 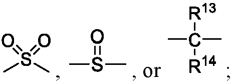 (2) J is none, X is -SO₂- or 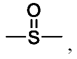 and Y is -S- or 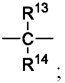 (3) J and X are both 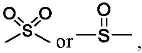 and Y is -S- or 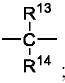 (4) J is none, X is 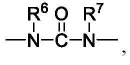 , and Y is -S- or

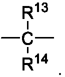

Furthermore, R, R¹, R², R³, R⁴ and R⁵ are all H or an aliphatic chain. Preferably, the aliphatic chain comprises an aliphatic hydrocarbon. More preferably, the aliphatic chain is an aliphatic hydrocarbon with 1 to 28 carbon atoms.

Furthermore, a is an integer of 1-3 (such as 1, 2 or 3, more preferably 1); b is an integer of 1-3 (such as 1, 2 or 3, more preferably 1); c is an integer of 1-3 (such as 1, 2 or 3, more preferably 1).

R, R¹~R¹⁴, and A₁~A₁₂ (*i.e.,* R, R¹, R², R³, R⁴, R⁵, R⁶, R⁷, R⁸, R⁹, R¹⁰, R¹¹, R¹², R¹³, R¹⁴, A₁, A₂, A₃, A₄, A₅, A₆, A₇, A₈, A₉, A₁₀, A₁₁, and A₁₂) are independently -H or an aliphatic hydrocarbon of 1-5 carbon atoms, i.e., independently -H, -CH₃, -CH₂CH₃, -CH₂CH₂CH₃, -CH(CH₃)CH₃, -CH₂CH₂CH₂CH₃, -CH(CH₃)CH₂CH₃, - CH₂CH(CH₃)CH₃, -C(CH₃)₃, -CH₂CH₂CH₂CH₂CH₃, -CH(CH₃)CH₂CH₂CH₃, -CH₂CH(CH₃)CH₂CH₃, - CH₂CH(CH₃)CH₂CH₃, -CH₂CH₂CH(CH₃)CH₃, -C(CH₃)₂CH₂CH₃, -CH₂C(CH₃)₂CH₃ or -CH(CH₃)CH(CH₃)CH₃, and R¹³~R¹⁴ cannot be H at the same time.

d is an integer of 0-14, e is an integer of 0-14, f is an integer of 1-15, and g is an integer of 1-4.

Furthermore, the structural formula of a labeled precursor of the fatty acid compound on the right side of general formula I is an isocyanate monomer 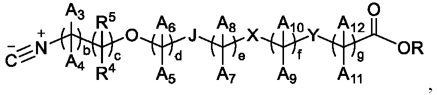 wherein J, X, Y, R⁴, R⁵, R, A₃~A₁₂, b, c, d, e, f and g are consistent with those defined in any of the above paragraphs, and are not repeated here; then the isocyanate monomer can be directly synthesized into general formula I by a one-step labeling method.

Furthermore, the structural formula of another labeling precursor of the fatty acid compound on the right side of general formula I is an isocyanate metal salt 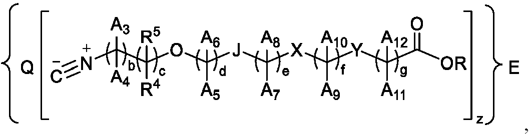 wherein Q is a metal cation, and the metal cation is preferably a copper ion, a cuprous ion, a calcium ion, a potassium ion, a sodium ion, a magnesium ion, an aluminum ion, and more preferably a copper ion or a cuprous ion; E is an anion, and the anion is preferably a tetrafluoroborate ion (BF₄⁻), a hexafluorophosphate ion (PF₆⁻), a trifluoroacetate ion (CF₃COO⁻), a perchlorate ion (ClO₄⁻), a fluoride ion, a chloride ion, a bromide ion, an iodide ion, and more preferably a tetrafluoroborate ion (BF₄⁻); J, X, Y, R⁴, R⁵, R, A₃~A₁₂, z, b, c, d, e, f and g are all consistent with those defined in any of the above paragraphs, and are not repeated here; then the isocyanate metal salt can be directly synthesized into general formula I by a one-step labeling method. Preferably, the precursor isocyanate metal salt is isocyanate copper salt 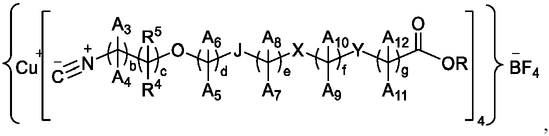 and then the isocyanate copper salt can be directly synthesized into general formula I by a one-step labeling method. The previous similar isocyano fatty acid structure is first labeled with an isocyanocarboxylate monomer precursor to form a carboxylate form of a rhenium or technetium complex, and then hydrolyzed under alkaline conditions to form the final rhenium or technetium complex carboxylic acid form. Therefore, the method of the present invention significantly shortens the intermediate links and time of labeling, especially overcomes the instability of the previous isocyanocarboxylate monomer labeling precursor, and the disadvantages of the hydrolysis step under alkaline conditions that damages the chemical stability of this type of technetium complex containing an isocyano structure.

Further, M is ^{99m}Tc or Re, z is an integer of 1-6, a and b are both 1, c is 1, R, R¹, R², R³, R⁴ and R⁵ are independently -H, -CH₃ or -CH₂CH₃, A₁-A₁₂ are all H, then the general formula **I** includes the following compounds:
①When d and e are both 0, J and X are both none, f is an integer of 1-15, g is an integer of 1-6, and Y is a sulfur atom (-S-), the formula I is the following general formula **II:** 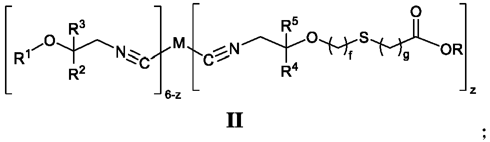
②When d and e are both 0, J and X are both none, f is an integer of 1-15, g is an integer of 1-6, and Y is sulfone 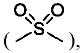 the formula I is the following general formula **III**: 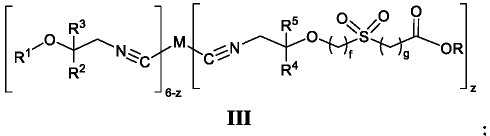
③When d is 0, J is none, e is an integer of 1-14, f is an integer of 1-15, g is an integer of 1-6, X is sulfone ( 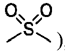 , and Y is a sulfur atom (-S-), the formula I is the following general formula **IV:** 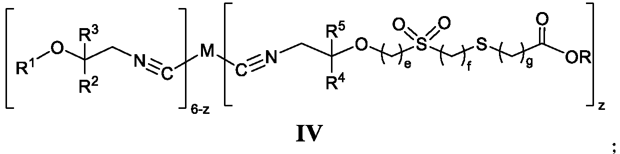
④When d and e are both integers of 1-14, f is an integer of 1-15, g is an integer of 1-6, J is sulfone 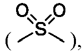 X is sulfone 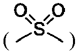 , and Y is a sulfur atom (-S-), the formula I is the following general formula **V:** 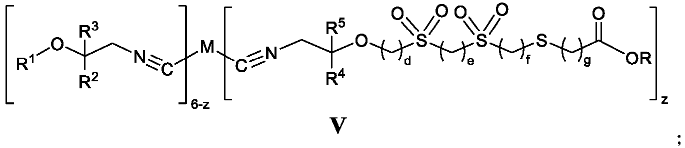
⑤When d is 0, J is none, e is an integer of 1-14, f is an integer of 1-15, g is an integer of 1-6, X is urea ( 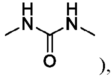 and Y is a sulfur atom (-S-), the formula I is the following general formula **VI:** 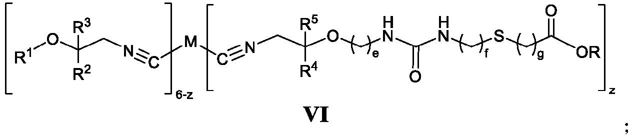
⑥When d and e are both 0, J and X are both none, f is an integer of 1-15, g is an integer of 1-6, and Y is 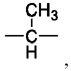 the formula I is the following general formula **VII:** 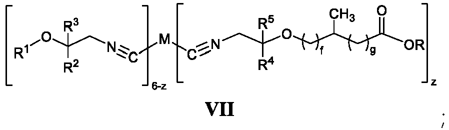
⑦When d and e are both 0, J and X are both none, f is an integer of 1-15, g is an integer of 1-6, and Y is 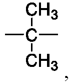 the formula I is the following general formula VIII**:**

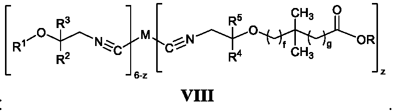

Furthermore, when z is an integer of 1-6, c is 1, a and b are both 1, d and e are both 0 (i.e., excluding J and X), f is an integer of 1-15, g is an integer of 1-6, and Y is -S-, Formula II includes but is not limited to the following compounds:

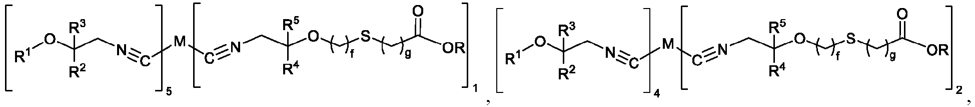

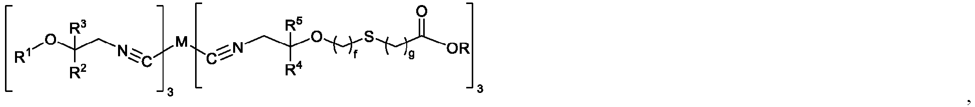

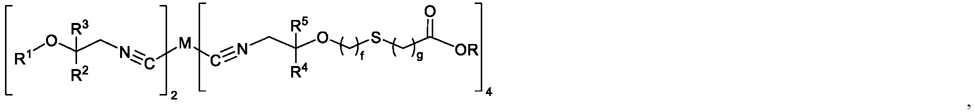

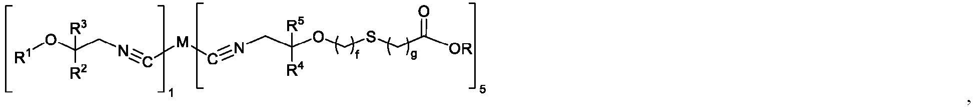

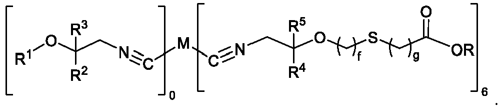

When z is an integer of 1-6, c is 1, a and b are both 1, d and e are both 0 (*i.e.,* J and X are not included), f is an integer of 1-15, g is an integer of 1-6, and Y is 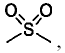 the formula III includes but is not limited to the following compounds:

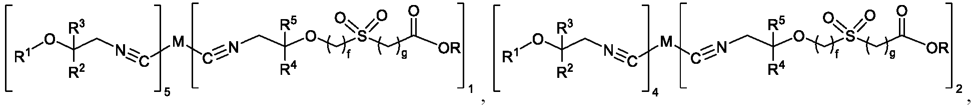

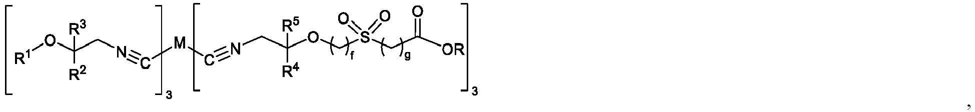

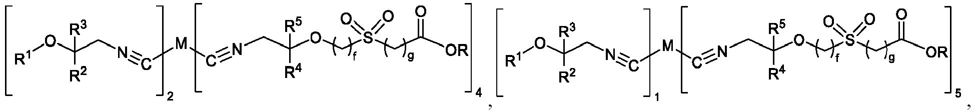

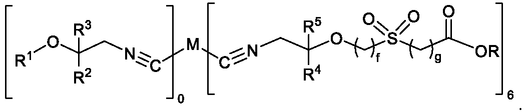

When z is an integer of 1-6, c is 1, a and b are both 1, d is 0 (i.e., does not contain J), e is an integer of 1-14, f is an integer of 1-15, g is an integer of 1-6, X is 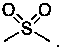 , and Y is -S-, Formula IV includes but is not limited to the following compounds:

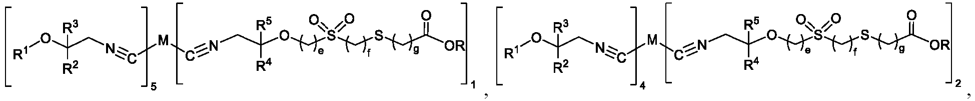

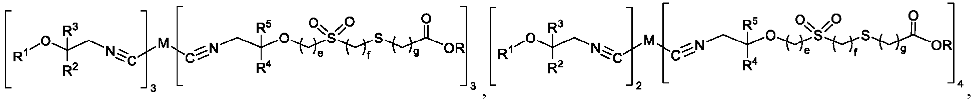

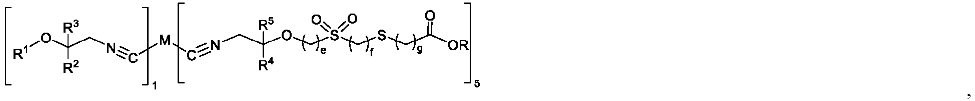

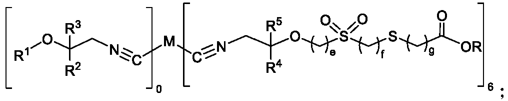

When z is an integer of 1-6, c is 1, a and b are both 1, d and e are both integers of 1-14, f is an integer of 1-15, g is an integer of 1-6, J is 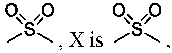 and Y is -S-, the general formula V includes but is not limited to the following compounds:

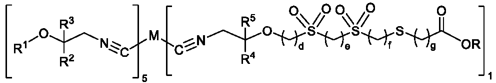

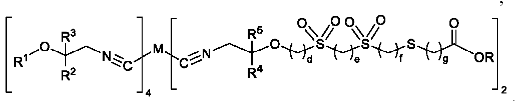

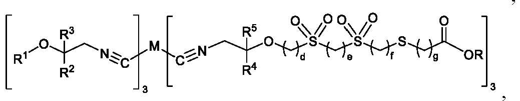

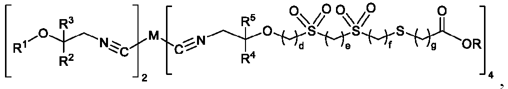

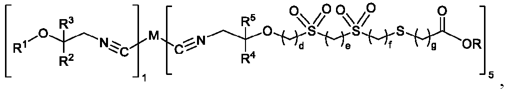

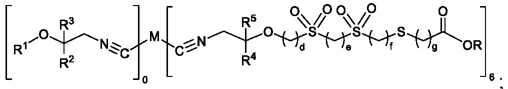

When z is an integer of 1-6, c is 1, a and b are both 1, d is 0 (i.e., does not contain J), e is an integer of 1-14, f is an integer of 1-15, g is an integer of 1-6, X is urea 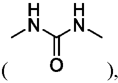 and Y is a sulfur atom (-S-), the general formula VI includes but is not limited to the following compounds:

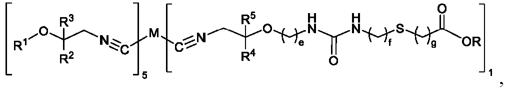

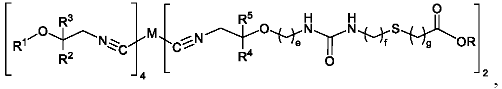

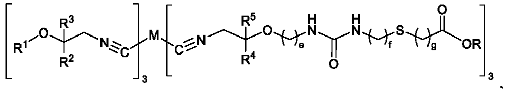

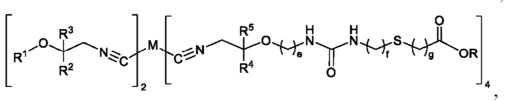

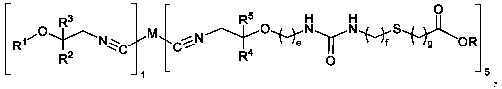

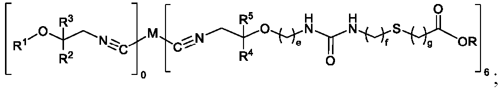

When z is an integer of 1-6, c is 1, a and b are both 1, d and e are both 0 (i.e., J and X are not included), f is an integer of 1-15, g is an integer of 1-6, and Y is, the general formula VII includes but is not limited to the following compounds:

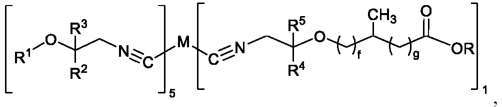

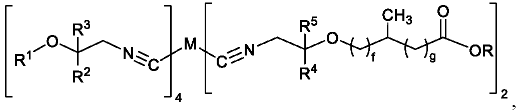

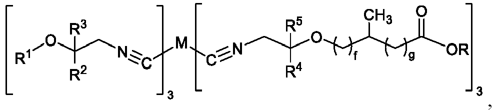

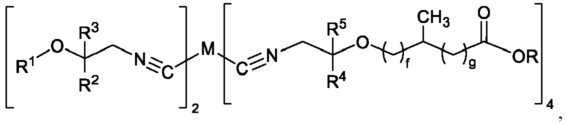

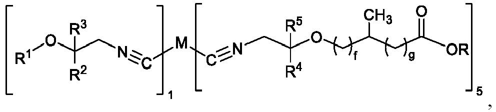

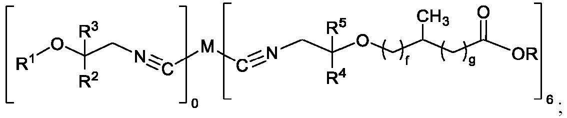

When z is an integer of 1-6, c is 1, a and b are both 1, d and e are both 0 (i.e., J and X are not included), f is an integer of 1-15, g is an integer of 1-6, and Y is 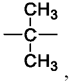 the general formula VIII includes but is not limited to the following compounds:

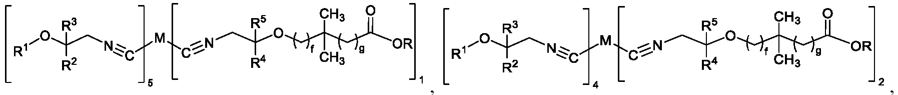

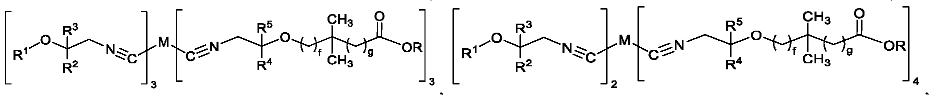

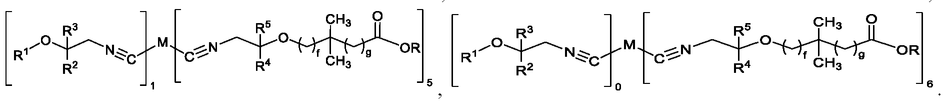

Wherein, M is labeled technetium ^{99m}Tc or Re, R, R¹, R², R³, R⁴ and R⁵ are all H or aliphatic hydrocarbons of 1-4 carbon atoms, d is an integer of 0-14, e is an integer of 0-14, f is an integer of 1-15, and g is an integer of 1-6.

Another inventive point of the present invention is to provide a precursor compound for preparing labeled fatty acid derivatives, wherein the precursor compound is an isocyanate monomer 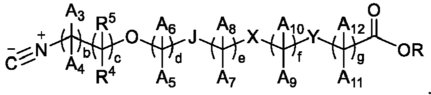 wherein J, X Y R⁵, R, A₃~A₁₂, z, b c d e, f and g are consistent with those defined in any of the above paragraphs, and will not be repeated here. The precursor can be synthesized into general formula I in one step.

Another inventive point of the present invention is to provide another precursor compound for preparing labeled fatty acid derivatives, wherein the precursor compound is an isocyanate metal salt 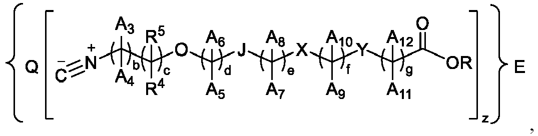 wherein Q is a metal cation, and the metal cation is preferably a copper ion, a cuprous ion, a calcium ion, a potassium ion, a sodium ion, a magnesium ion, an aluminum ion, and more preferably a copper ion or a cuprous ion; E is an anion, and the anion is preferably a tetrafluoroborate ion (BF₄⁻), a hexafluorophosphate ion (PF₆⁻), a trifluoroacetate ion (CF₃COO⁻), a perchlorate ion (ClO₄⁻), a fluoride ion, a chloride ion, a bromide ion, an iodide ion, and more preferably a tetrafluoroborate ion (BF₄⁻); J, X, Y, R⁴, R⁵, A₃~A₁₂, z, b, c, d, e, f and g are all consistent with those defined in any of the above paragraphs, and will not be repeated here. The general formula I can be synthesized in one step from the precursor.

Another aspect of the present invention is to provide a use of the labeled fatty acid derivatives described in any of the above paragraphs in a myocardial imaging agent.

The last invention of the present invention is to provide a myocardial imaging agent, which includes the general formula I described in any of the above paragraphs.

The main beneficial effects of the present application are as follows: the present invention is a completely new structure, which creatively adds a new and unique group that has never existed before to the structure of isocyanate-containing fatty compounds, that is, at least one structure among J, X and Y in the general formula I is added, and the other partial structures are modified at the same time, becoming a completely new type of structure. In the fatty acid compound, when at least one ligand terminal in the new structure is a carboxylic acid or ester group (that is, when z is not equal to 0), and a simple sulfur atom or oxygen atom or sulfone group or similar group is added at a suitable position away from the carboxylic acid or ester group, or on this basis, a sulfone group, urea bridge or similar structure is added at a suitable position, the fatty acid imaging agent can be taken up in the myocardium with high absorption, long retention time, low background in the lung, blood and liver, and the water solubility of the imaging agent can be improved.

Specifically, using the compounds of the present application, the lung background and blood background are very low throughout the process; the myocardial absorption is weak at the beginning, and the liver background is high; as time goes on, the liver background gradually weakens, while the myocardial absorption gradually increases; especially about 60 min after the tail vein injection, the liver uptake is basically not observed, while the myocardial absorption is the strongest at this time, and the myocardial imaging is very obvious; thereafter, as the time after the intravenous injection is further extended, the myocardial absorption gradually weakens, and even basically disappears. This reflects the characteristics of myocardial metabolic imaging agents. Compared with traditional myocardial perfusion imaging agents, such as ^{99m}Tc-MIBI (whose myocardial absorption is always strong), this type of imaging agent in the present invention may be able to better reflect the vitality and metabolic state of the myocardium, and be better used for the diagnosis of heart disease and the judgment of myocardial cell survival, so that it can be used as a myocardial fatty acid metabolism imaging agent, which has important clinical application prospects.

### BRIEF DESCRIPTION OF DRAWINGS

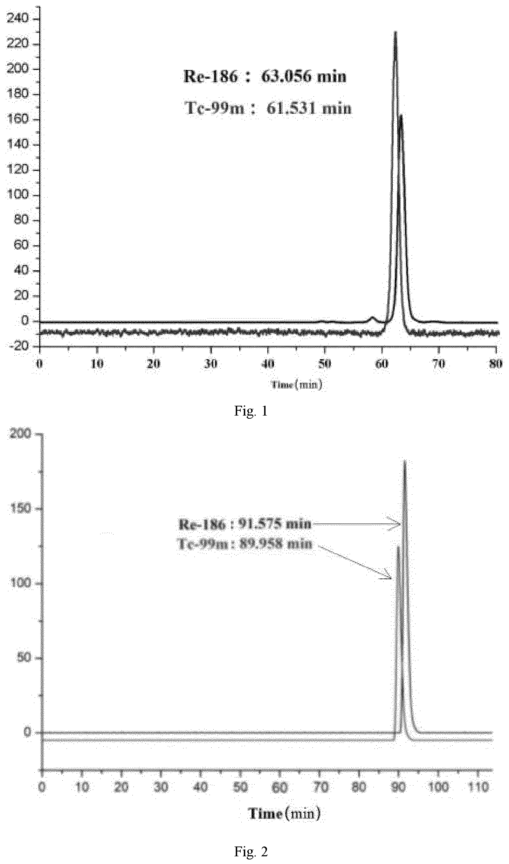

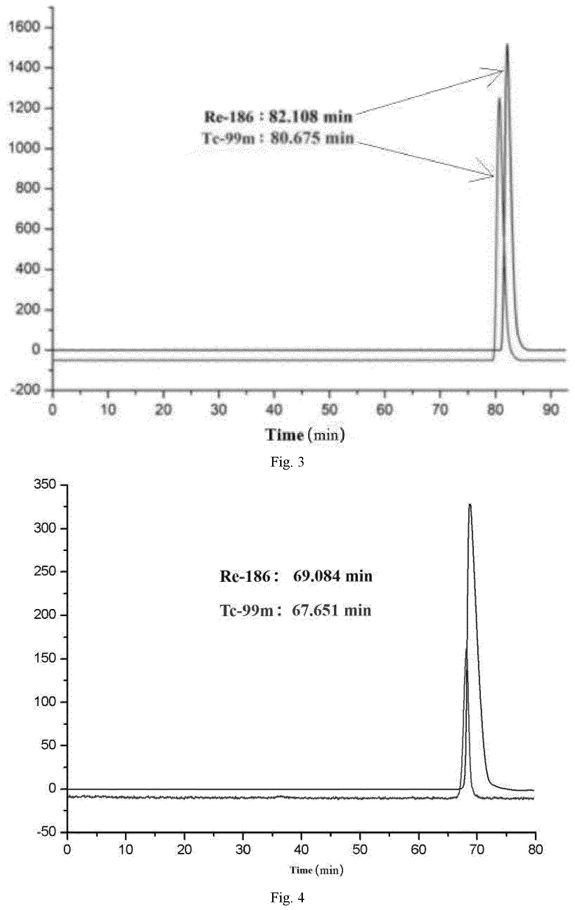

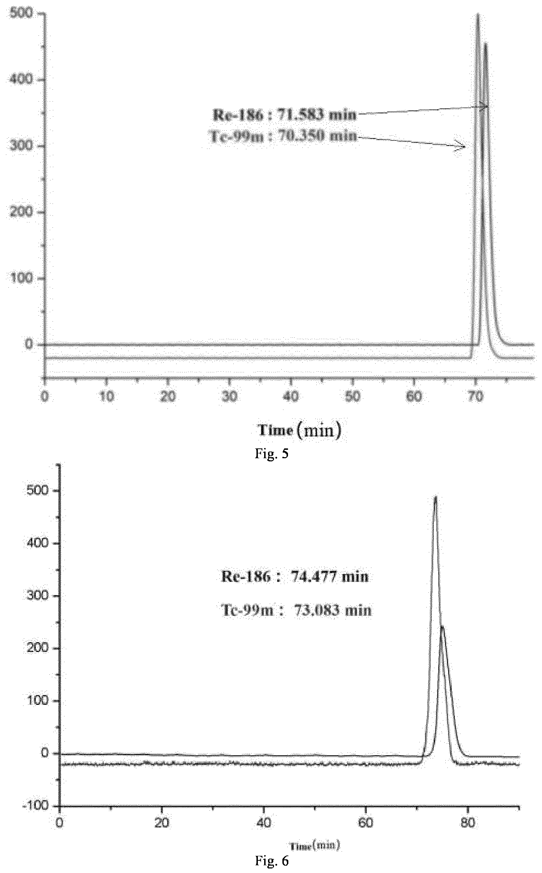

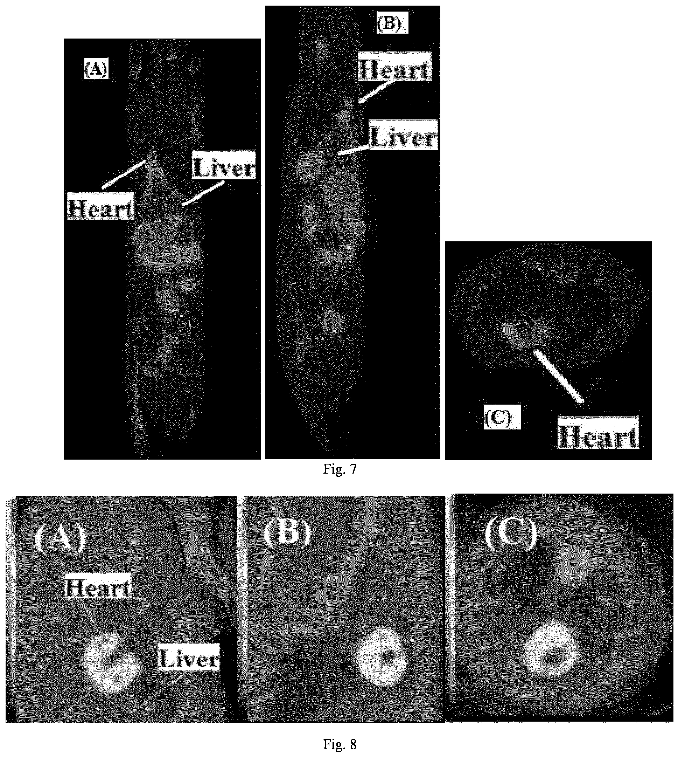

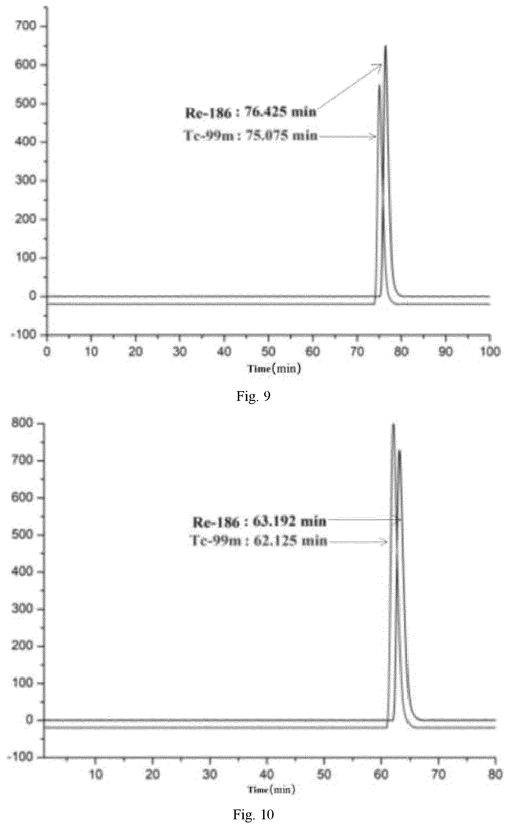

Fig. 1 is a HPLC co-injection spectrum of the ^{99m}Tc radioactive complex **78** described in Example 1 of the present invention and its corresponding Re complex **69;** Fig. 2 is a HPLC co-injection spectrum of the ^{99m[}Tc radioactive complex **79** described in Example 1 of the present invention and its corresponding Re complex **71;** Fig. 3 is a HPLC co-injection spectrum of the ^{99m}Tc radioactive complex **80** described in Example 1 of the present invention and its corresponding Re complex **72;** Fig. 4 is a HPLC co-injection spectrum of the ^{99m}Tc radioactive complex 81 described in Example 1 of the present invention and its corresponding Re complex **73;** Fig. 5 is a HPLC co-injection spectrum of the ^{99m}Tc radioactive complex **82** described in Example 1 of the present invention and the corresponding Re complex **74;** Fig. 6 is a HPLC co-injection spectrum of the ^{99m}Tc radioactive complex **83** described in Example 1 of the present invention and the corresponding Re complex **75;** Fig. 7 is a representative dynamic SPECT/CT image of the radioactive ^{99m}Tc complex **79** described in Example 3 of the present invention in female SD rats (A, B and C of Fig. 7 are SPECT/CT images of the ^{99m}Tc complex **79** in female SD rats at 56-64 min post-injection, respectively). Fig. 8 is a representative dynamic SPECT/CT image of the radioactive ^{99m}Tc complex **80** described in Example 3 of the present invention in female SD rats (A, B and C of Fig. 8 are respectively the coronal, sagittal and cross-sectional images of the dynamic SPECT/CT imaging of the ^{99m}Tc complex **80** at 66-72 min post-injection in the female SD rat); Fig. 9 is a HPLC co-injection spectrum of the ^{99m}Tc radioactive complex **130** described in Example 6 of the present invention and the corresponding Re complex **127;** Fig. 10 is a HPLC co-injection spectrum of the ^{99m}Tc radioactive complex **158** described in Example 10 of the present invention and the corresponding Re complex **157.** In Figs. 1, 4 and 6, the compound with a short time (*i.e.,* an early peak time) corresponds to the peak on the left, and the compound with a long time (*i.e.,* a late peak time) corresponds to the peak on the right.

### DETAILED DESCRIPTION

The technical solutions in the embodiments of the present invention are described clearly and completely below. Obviously, the described embodiments are only part of the embodiments of the present invention, not all of the embodiments. The detailed description of the embodiments of the present invention provided below is not intended to limit the scope of the invention claimed for protection, but merely represents selected embodiments of the present invention. Based on the embodiments of the present invention, all other embodiments obtained by ordinary method personnel in the field without creative work are within the scope of protection of the present invention.

The specific synthesis process of the compounds in II, III, IV, V, VI, VII, and VIII separately defined in the present invention is introduced below. Since the synthesis process cannot be exhaustively listed for each protected compound, the following method can be referred to for those not listed, which is completely achievable for those skilled in the art. In addition, due to the length of the article, this application only describes the effects of several structures, but the structures not listed also have similar effects.

### Example 1

The organic synthesis routes and methods of the labeled precursors **59-68** of the typical compounds of the general formula **II** in the present application, as well as the labeling routes and methods of the Re (rhenium-186) complexes **69-77** of some typical compounds and some of their corresponding radioactive ^{99m}Tc (technetium-99m) complexes **78-83** are selected, and the cases where f in the general formula **II** is 2, 3, 4, 5, 6, 7, 11, and g is 4; the case where f is 2 and g is 6; the case where f is 3 and g is 5; and the case where f is 6 and g is 2 are listed as follows:
(1) The synthesis and labeling routes are as follows: 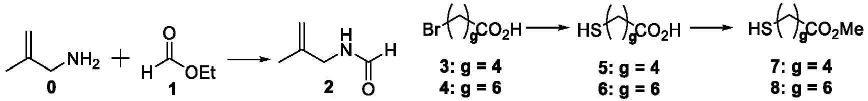 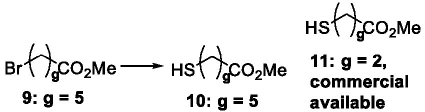 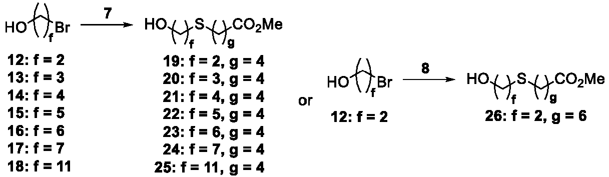 or 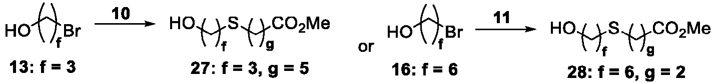 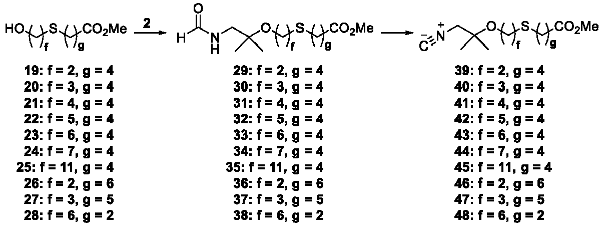 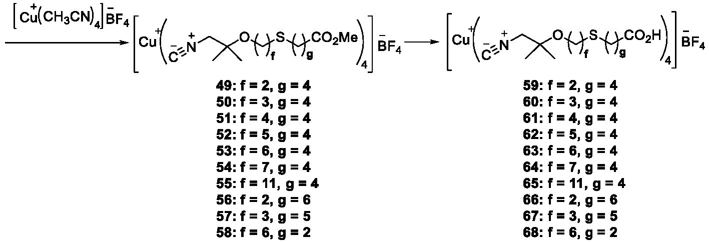 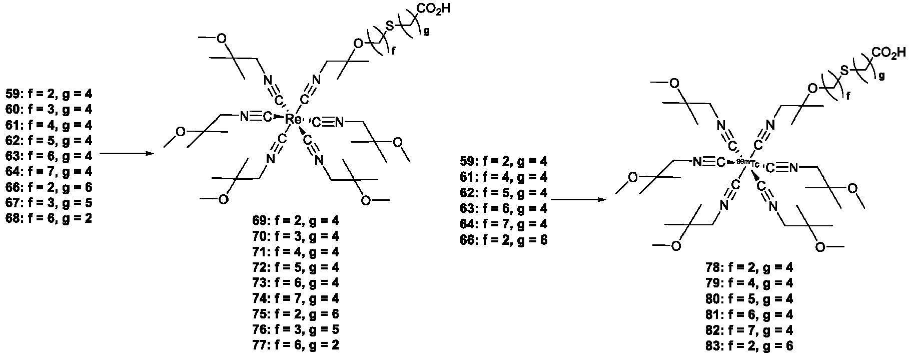
(2) The synthesis and labeling process is as follows:
   Synthesis of intermediate **2:** Add raw material **1** (48.7460 g) to starting material 0 (39.0000 g) at 0 °C. Then, stir at 60 °C for 3 h. Concentrate the reaction solution in vacuo to obtain 50.1200 g of concentrated solution to obtain intermediate 2. ¹H NMR (400MHz,CDCl3 , δ ppm):8.23(s,1H),6.52(brs,1H),4.86(s,2H),3.83(d,J= 7.5Hz,2H),1.75(s,3H).
   Synthesis of intermediate **5:** Raw material **3** (100.0000 g) was dissolved in anhydrous ethanol, and then thiourea (63.0720 g) was added, and the mixture was refluxed and stirred for 20 h. Then, the mixture was cooled to room temperature and the ethanol was removed by rotary evaporation. Then, 7.5 mol/L aqueous sodium hydroxide solution (1001.6756 mL) was added to the obtained mother liquor, and the mixture was heated at 90 °C for 16 h. After cooling, 2 mol/L aqueous sulfuric acid solution was added dropwise thereto, and the pH value of the mixture was adjusted to about 2. The mixture was extracted twice with dichloromethane, and the combined organic phases were dried over anhydrous sodium sulfate to give intermediate 5 (58.6516 g). ¹H NMR (400 MHz, CDCl₃): δ 11.09 (brs, 1H), 2.55 (q, *J =* 7.4 Hz, 2H), 2.38 (t, *J =* 7.0 Hz, 2H), 1.64-1.79 (m, 4H), 1.37 (t, *J =* 7.8 Hz, 1H).
   Synthesis of intermediate **6:** For specific operations, refer to "Synthesis of intermediate **5".** Intermediate **12** (47.9633 g) was obtained from raw material **4** (100.0000 g). ¹H NMR (400 MHz, CDCl₃): δ 11.09 (brs, 1H), 2.53 (q, *J =* 7.4 Hz, 2H), 2.36 (t, *J =* 7.4 Hz, 2H), 1.58-1.69 (m, 4H), 1.31-1.45 (m, 5H).
   Synthesis of intermediate **7:** Intermediate **5** (58.6520 g) was dissolved in anhydrous methanol, and then p-toluenesulfonic acid (7.5265 g) was added, and the mixture was refluxed and stirred overnight. After cooling, it was concentrated *in vacuo,* quenched by adding saturated sodium bicarbonate aqueous solution, extracted with ethyl acetate, dried, and concentrated *in vacuo* to obtain intermediate 7 (45.2640 g).
   Synthesis of intermediate **8:** For specific operation, refer to "Synthesis of intermediate **7".** Intermediate **12** (42.5464 g) was obtained from raw material **6** (47.9633 g) and p-toluenesulfonic acid (5.0905 g). ¹H NMR (400 MHz, CDCl₃): δ 11.09 (brs, 1H), 2.53 (q, *J* = 7.4 Hz, 2H), 2.36 (t, *J* = 7.4 Hz, 2H), 1.58-1.69 (m, 4H), 1.31-1.45 (m, 5H).
   Synthesis of intermediate **10:** The raw material **9** (30.0000 g) was dissolved in anhydrous ethanol, and then thiourea (13.2158 g) was added, and the mixture was refluxed and stirred for 18 h. Then, after the mixture was cooled to room temperature, the ethanol was removed by rotary evaporation. Then, an aqueous solution of sodium hydroxide (7.5670 g) was added to the obtained mother liquor, and the mixture was refluxed and stirred for 1 h. After cooling, the mixture was extracted twice with dichloromethane, and the combined organic phases were dried over anhydrous sodium sulfate to obtain intermediate **10** (7.0458 g). ¹H NMR (400 MHz, CDCl₃): δ 3.59 (s, 3H), 2.46 (t, *J =* 7.2 Hz, 2H), 2.25 (t, *J =* 7.5 Hz, 2H), 1.52-1.61 (m, 4H), 1.32-1.39 (m, 2H).
   Synthesis of intermediate **19:** Raw material **12** (12.0000 g), intermediate **7** (17.0790 g) and anhydrous potassium carbonate (16.8546 g) were stirred at room temperature overnight. The mixture was filtered, concentrated under reduced pressure, and separated by silica gel column chromatography to obtain intermediate **19** (14.6364 g). ¹H NMR (400 MHz, CDCl₃): δ 3.72 (t, *J* = 6.4 Hz, 2H), 3.67 (s, 3H), 2.70 (t, *J* = 6.5 Hz, 2H), 2.55 (t*, J =* 7.2 Hz, 2H), 2.34 (t, *J =* 7.3 Hz, 2H), 1.67-1.77 (m, 2H), 1.57-1.67 (m, 2H).
   Synthesis of intermediate **20:** For specific operation, refer to "Synthesis of intermediate **19".** Intermediate **20** (13.3987 g) was obtained from raw material **13** (15.0000 g), intermediate 7 (19.1953 g) and anhydrous potassium carbonate (18.9431 g). ¹H NMR (400 MHz, CDCl₃): δ 3.69 (t, *J* = 6.2 Hz, 2H), 3.67 (s, 3H), 2.61 (t, *J* = 7.3 Hz, 2H), 2.53 (t, *J =* 7.2 Hz, 2H), 2.34 (t, *J =* 7.3 Hz, 2H), 1.78-1.86 (m, 2H), 1.68-1.77 (m, 2H), 1.57-1.67 (m, 2H).
   Synthesis of intermediate **21:** For specific operation, refer to "Synthesis of intermediate **19".** Intermediate **21** (6.6741 g) was obtained from raw material **14** (8.8499 g), intermediate 7 (10.2868 g) and anhydrous potassium carbonate (10.1516 g). ¹H NMR (400 MHz, CDCl₃): δ 3.66 (s, 3H), 3.61 (t, *J* = 5.6 Hz, 2H), 2.50-2.55 (m, 4H), 2.34 (t, *J =* 7.2 Hz, 2H), 1.57-1.76 (m, 8H).
   Synthesis of intermediate **22:** For specific operation, refer to "Synthesis of intermediate **19".** Intermediate **22** (24.6306 g) was obtained from raw material **15** (15.0000 g), intermediate **7** (23.9565 g) and anhydrous potassium carbonate (23.6417 g). ¹H NMR (400 MHz, CDCl₃): δ 3.67 (s, 3H), 3.37 (t, *J* = 8.6 Hz, 2H), 3.18 (t, *J* = 7.3 Hz, 1H), 2.29-2.33 (m, 4H), 2.15 (t, *J* = 5.4 Hz, 2H), 1.48-1.54 (m, 2H), 1.34-1.41 (m, 6H), 1.22-1.28 (m, 2H).
   Synthesis of intermediate **23:** For specific operation, refer to "Synthesis of intermediate **19".** Intermediate **23** (18.0194 g) was obtained from raw material **16** (15.0000 g), intermediate 7 (14.7344 g) and anhydrous potassium carbonate (14.5408 g). ¹H NMR (400 MHz, CDCl₃, CDCl₃): δ 3.67 (s, 3H), 3.61 (t, *J* = 5.2 Hz, 2H), 2.49-2.53 (m, 4H), 2.45 (t, *J =* 7.3 Hz, 1H), 2.34 (t, *J* = 7.3 Hz, 2H), 1.69-1.76 (m, 2H), 1.53-1.65 (m, 6H), 1.37-1.46 (m, 4H).
   Synthesis of intermediate **24:** For specific operation, refer to "Synthesis of intermediate **19".** Intermediate **24** (12.8443 g) was obtained from raw material **17** (23.7877 g), intermediate **7** (23.3666 g) and anhydrous potassium carbonate (23.0595 g). ¹H NMR (400 MHz, CDCl₃): δ 3.67 (s, 3H), 3.64 (t, *J* = 6.6 Hz, 2H), 2.51 (q, *J* = 7.0 Hz, 4H), 2.34 (t, *J =* 7.3 Hz, 2H), 1.70-1.76 (m, 2H), 1.54-1.65 (m, 6H), 1.30-1.43 (m, 6H).
   Synthesis of intermediate **25:** For specific operations, refer to "Synthesis of intermediate **19".** Intermediate **25** (19.9610 g) was obtained from raw material **18** (34.3875 g), intermediate **7** (24.3474 g) and anhydrous potassium carbonate (24.0274 g). ¹H NMR (400 MHz, CDCl₃): δ 3.67 (s, 3H), 3.64 (t, *J=* 6.6 Hz, 2H), 2.48-2.53 (m, 4H), 2.33 (t, *J =* 7.2 Hz, 2H), 1.70-1.77 (m, 2H), 1.53-1.65 (m, 6H), 1.29-1.39 (m, 15H).
   Synthesis of intermediate **26:** For specific operations, refer to "Synthesis of intermediate **19".** Intermediate **26** (5.4869 g) was obtained from raw material **12** (5.7779 g), intermediate **8** (9.7797 g) and anhydrous potassium carbonate (8.1154 g). ¹H NMR (400 MHz, CDCl₃): δ 3.72 (t, *J* = 6.2 Hz, 2H), 3.67 (s, 3H), 2.71 (t, *J* = 6.2 Hz, 2H), 2.52 (t, *J =* 7.4 Hz, 2H), 2.31 (t, *J =* 7.5 Hz, 2H), 1.54-1.67 (m, 4H), 1.30-1.44 (m, 4H).
   Synthesis of intermediate **27:** For specific operations, refer to "Synthesis of intermediate **19".** Intermediate 27 (5.7569 g) was obtained from raw material **13** (10.0000 g), intermediate **10** (14.0082 g) and anhydrous potassium carbonate (12.6287 g). ¹H NMR (400 MHz, CDCl₃): δ 3.66 (t, *J* = 6.1 Hz, 2H), 3.60 (s, 3H), 2.55 (t, *J* = 7.1 Hz, 2H), 2.46 (t, *J =* 7.3 Hz, 2H), 2.25 (t, *J =* 7.5 Hz, 2H), 1.72-1.81 (m, 2H), 1.50-1.62 (m, 4H), 1.30-1.40 (m, 2H).
   Synthesis of intermediate **28:** For specific operations, refer to "Synthesis of intermediate **19".** Intermediate **28** (9.9812 g) was obtained from raw material **16** (10.0260 g), intermediate **11** (7.9847 g) and anhydrous potassium carbonate (9.7191 g). ¹H NMR (400 MHz, CDCl₃): δ 3.84 (brs, 1H), 3.70 (s, 3H), 3.59 (t, *J* = 6.6 Hz, 2H), 2.77 (t, *J* = 7.4 Hz, 2H), 2.61 (t, *J =* 7.4 Hz, 2H), 2.53 (t, *J =* 7.4 Hz, 2H), 1.51-1.63 (m, 4H), 1.33-1.45 (m, 4H).
   Synthesis of intermediate 29: Intermediate 2 (5.0308 g), intermediate **19** (14.6364 g), and mercuric acetate (20.0543 g) were dissolved in dichloromethane, and the mixture was stirred at room temperature overnight, filtered, and the filtrate was concentrated in vacuo. The concentrate was dissolved in methanol, sodium borohydride (2.0350 g) was added, and then stirred at room temperature for 60 min, filtered, and the filtrate was concentrated in vacuo. The concentrate was redissolved in dichloromethane, filtered again and concentrated in vacuo. Silica gel column chromatography was used to separate and obtain relatively pure intermediate **29** (3.4613 g).
   Synthesis of intermediate **30:** For specific operations, see "Synthesis of intermediate **29".** Relatively pure intermediate **30** (1.2856 g) was obtained from intermediate **2** (4.2922 g), intermediate **20** (13.3987 g), mercuric acetate (17.1099 g) and sodium borohydride (1.7363 g).
   Synthesis of intermediate **31:** For specific operations, refer to "Synthesis of intermediate **29".** Relatively pure intermediate **31** (1.4626 g) was obtained from intermediate **2** (2.0019 g), intermediate **21** (6.6741 g), mercuric acetate (7.9800 g) and sodium borohydride (0.8098 g). HRMS: calcd 342.1715 (M+Na)⁺, found 342.1710 (M+Na)⁺.
   Synthesis of intermediate **32:** For specific operations, see "Synthesis of intermediate **29".** Relatively pure intermediate **32** (2.4097 g) was obtained from intermediate 2 (4.6169 g), intermediate **22** (16.3720 g), mercuric acetate (18.4044 g) and sodium borohydride (1.8676 g).
   Synthesis of intermediate **33:** For specific operations, see "Synthesis of intermediate **29".** Relatively pure intermediate **33** (3.7046 g) was obtained from intermediate **2** (4.7944 g), intermediate **23** (18.0194 g), mercuric acetate (19.1121 g) and sodium borohydride (1.9394 g).
   Synthesis of intermediate **34:** For specific operations, refer to "Synthesis of intermediate **29".** Relatively pure intermediate **34** (1.8431 g) was obtained from intermediate **2** (1.6048 g), intermediate **24** (6.3720 g), mercuric acetate (6.3971 g) and sodium borohydride (0.6492 g). HRMS: calcd 362.2365 (M+H)⁺, found 362.2484 (M+H)⁺, calcd 384.2184 (M+Na)⁺, found 384.2306 (M+Na)⁺.
   Synthesis of intermediate **35:** For specific operations, refer to "Synthesis of intermediate **29".** Relatively pure intermediate **35** (1.3249 g) was obtained from intermediate **2** (1.3669 g), intermediate **25** (6.5880 g), mercuric acetate (5.4488 g) and sodium borohydride (0.5529 g). HRMS: calcd 418.2991 (M+H)⁺, found 418.2993 (M+H)⁺, calcd 440.2810 (M+Na)⁺, found 440.2809 (M+Na)⁺.
   Synthesis of intermediate **36:** For specific operations, see "Synthesis of intermediate **29".** Relatively pure intermediate **36** (1.3966 g) was obtained from intermediate **2** (1.6458 g), intermediate **26** (5.4869 g), mercuric acetate (6.5605 g) and sodium borohydride (0.6657 g).
   Synthesis of intermediate **37:** For specific operations, refer to "Synthesis of intermediate **29".** Relatively pure intermediate **37** (2.9145 g) was obtained from intermediate **2** (1.7267 g), intermediate **27** (5.7569 g), mercuric acetate (6.8834 g) and sodium borohydride (0.6985 g).
   Synthesis of intermediate **38:** For specific operations, see "Synthesis of intermediate **29".** Relatively pure intermediate **38** (2.9414 g) was obtained from intermediate **2** (2.9938 g), intermediate **28** (9.9812 g), mercuric acetate (11.9342 g) and sodium borohydride (1.2110 g).
   Synthesis of isocyanomethyl ester intermediate **39:** To a solution of intermediate **29** (3.4613 g) in dichloromethane was add triethylamine (3.3293 g) and phosphorus oxychloride (2.0032 g). And the resulted mixture was stirred at room temperature for 20 to 30 min. It was quenched with 20% potassium carbonate aqueous solution, extracted with dichloromethane, concentrated *in vacuo,* and separated by silica gel column chromatography to obtain isocyanomethyl ester intermediate **39** (0.4163 g). ¹H NMR (400 MHz, CDCl₃): δ 3.67 (s, 3H), 3.55 (t, *J* = 6.8 Hz, 2H), 3.38 (s, 2H), 2.66 (t, *J* = 6.8 Hz, 2H), 2.59 (t, *J =* 7.2 Hz, 2H), 2.34 (t, *J* = 7.3 Hz, 2H), 1.70-1.75 (m, 2H), 1.60-1.66 (m, 2H), 1.29 (s, 6H).
   Synthesis of isocyanomethyl ester intermediate 40: For specific operations, refer to "Synthesis of isocyanomethyl ester intermediate 39". Isocyanomethyl ester intermediate 40 (0.3249 g) was obtained from intermediate 30 (1.2856 g), triethylamine (1.1798 g) and phosphorus oxychloride (0.7099 g). ¹H NMR (400 MHz, CDCl₃): δ 3.67 (s, 3H), 3.46 (t, *J =* 6.0 Hz, 2H), 3.37 (s, 2H), 2.60 (t, *J=* 7.1 Hz, 2H), 2.52 (t, *J* = 7.2 Hz, 2H), 2.34 (t, *J=* 7.3 Hz, 2H), 1.77-1.85 (m, 2H), 1.69-1.77 (m, 2H), 1.60-1.66 (m, 2H), 1.28 (s, 6H).
   Synthesis of isocyanomethyl ester intermediate **41:** For specific operations, refer to "Synthesis of isocyanomethyl ester intermediate **39".** Isocyanomethyl ester intermediate **41** (0.6590 g) was obtained from intermediate **31** (1.4626 g), triethylamine (1.2833 g) and phosphorus oxychloride (0.7721 g). ¹H NMR (400 MHz, CDCl₃): δ 3.67 (s, 3H), 3.33-3.41 (m, 4H), 2.48-2.57 (m, 4H), 2.34 (t, *J =* 7.3 Hz, 2H), 1.70-1.78 (m, 2H), 1.60-1.67 (m, 6H), 1.27 (s, 6H).
   Synthesis of isocyanomethyl ester intermediate **42:** For the specific operation, refer to "Synthesis of isocyanomethyl ester intermediate **39".** Isocyanomethyl ester intermediate **42** (0.641g). ¹H NMR (400 MHz, CDCl₃): δ 3.67 (s, 3H), 3.33-3.39 (m, 4H), 2.48-2.54 (m, 4H), 2.34 (t, *J =* 7.3 Hz, 2H), 1.69-1.76 (m, 2H), 1.53-1.64 (m, 6H), 1.42-1.49 (m, 2H), 1.27 (s, 6H).
   Synthesis of isocyanomethyl ester intermediate **43:** For specific operations, refer to "Synthesis of isocyanomethyl ester intermediate **39".** Isocyanomethyl ester intermediate **43** (1.0299 g) was obtained from intermediate 33 (3.7046 g), triethylamine (2.9881 g) and phosphorus oxychloride (1.7979 g). ¹H NMR (400 MHz, CDCl₃): δ 3.67 (s, 3H), 3.32-3.39 (m, 4H), 2.47-2.55 (m, 4H), 2.34 (t, *J =* 7.3 Hz, 2H), 1.69-1.77 (m, 2H), 1.51-1.65 (m, 6H), 1.35-1.44 (m, *J =* 4.5, 3.4 Hz, 4H), 1.27 (s, 6H).
   Synthesis of isocyanomethyl ester intermediate **44:** For specific operations, refer to "Synthesis of isocyanomethyl ester intermediate **39".** Isocyanomethyl ester intermediate **44** (0.8140 g) was obtained from intermediate **34** (1.8431 g), triethylamine (1.4289 g) and phosphorus oxychloride (0.8598 g). ¹H NMR (400 MHz, CDCl₃): δ 3.67 (s, 3H), 3.31-3.38 (m, 4H), 2.50 (q, *J* = 7.3 Hz, 4H), 2.33 (t, *J =* 7.3 Hz, 2H), 1.69-1.77 (m, 2H), 1.50-1.64 (m, 6H), 1.31-1.42 (m, 6H), 1.27 (s, 6H).
   Synthesis of isocyanomethyl ester intermediate **45:** For specific operations, refer to "Synthesis of isocyanomethyl ester intermediate **39".** Isocyanomethyl ester intermediate **45** (0.9372 g) was obtained from intermediate 35 (1.3249 g), triethylamine (1.4289 g) and phosphorus oxychloride (0.8598 g). ¹H NMR (400 MHz, CDCl₃): δ 3.67 (s, 3H), 3.31-3.39 (m, 4H), 2.46-2.54 (m, 4H), 2.33 (t, *J =* 7.3 Hz, 2H), 1.42-1.81 (m, 9H), 1.27-1.41 (m, 13H), 1.27 (s, 6H).
   Synthesis of isocyanomethyl ester intermediate **46:** For specific operations, refer to "Synthesis of isocyanomethyl ester intermediate **39".** Isocyanomethyl ester intermediate **46** (0.4487 g) was obtained from intermediate 36 (1.3966 g), triethylamine (1.2254 g) and phosphorus oxychloride (0.7373 g). ¹H NMR (400 MHz, CDCl₃): δ 3.67 (s, 3H), 3.55 (t, *J* = 6.8 Hz, 2H), 3.38 (s, 2H), 2.66 (t, *J =* 6.9 Hz, 2H), 2.56 (t, *J* = 7.4 Hz, 2H), 2.31 (t, *J =* 7.5 Hz, 2H), 1.56-1.66 (m, 4H), 1.33-1.43 (m, 4H), 1.29 (s, 6H).
   Synthesis of isocyanomethyl ester intermediate **47:** For specific operations, refer to " Synthesis of isocyanomethyl ester intermediate **39** ". Isocyanomethyl ester intermediate **47** (0.9000 g) was obtained from intermediate 37 (2.9145 g), triethylamine (2.5572 g) and phosphorus oxychloride (1.5386 g). ¹H NMR (400 MHz, CDCl₃): δ 3.46 (t, *J =* 6.1 Hz, 2H), 3.37 (s, 2H), 2.60 (t, *J =* 7.1 Hz, 2H), 2.51 (t, *J =* 7.4 Hz, 2H), 2.32 (t, *J =* 7.5 Hz, 2H), 1.85 - 1.77 (m, 2H), 1.67 - 1.58 (m, 4H), 1.46 - 1.39 (m, 2H), 1.28 (s, 6H).
   Synthesis of isocyanomethyl ester intermediate **48:** For specific operations, refer to" Synthesis of isocyanomethyl ester intermediate **39".** Isocyanomethyl ester intermediate **48** (1.2494 g) was obtained from intermediate 38 (2.9414 g), triethylamine (2.5808 g) and phosphorus oxychloride (1.5528 g). ¹H NMR (400 MHz, CDCl₃): δ 3.70 (s, 3H), 3.31-3.39 (m, 4H), 2.78 (t, *J =* 7.4 Hz, 2H), 2.61 (t, *J* = 7.4 Hz, 2H), 2.53 (t, *J* = 7.4 Hz, 2H), 1.51-1.62 (m, 4H), 1.34-1.42 (m, 4H), 1.27 (s, 6H).
   Synthesis of copper salt methyl ester intermediate **49:** The isocyanomethyl ester intermediate **39** (0.4163 g) and tetra(acetonitrile)copper(I) tetrafluoroborate (0.1197 g) were stirred in dichloromethane at room temperature for 30-60 min, filtered, concentrated *in vacuo,* and separated by silica gel column chromatography to obtain relatively pure copper salt methyl ester intermediate **49** (0.1560 g). In addition, the isocyanomethyl ester intermediate **39** (1.00 equiv.) and cuprous chloride (0.25 equiv.) were stirred in a mixed solvent of ethanol and dichloromethane at room temperature for 15 min, and then ammonium tetrafluoroborate (1.06 equiv.) was added and stirred at 60 °C for 15 min, filtered, concentrated *in vacuo,* and separated by silica gel column chromatography to obtain relatively pure copper salt methyl ester intermediate **49.**
   Synthesis of copper methyl ester intermediate **50:** For specific operations, refer to "Synthesis of copper methyl ester intermediate **49".** Relatively pure copper methyl ester intermediate **50** (0.1799 g) was obtained from isocyanomethyl ester intermediate **40** (0.3249 g) and tetra(acetonitrile)copper(I) tetrafluoroborate (0.0889 g).
   Synthesis of copper methyl ester intermediate **51:** For the specific operation, refer to "Synthesis of copper methyl ester intermediate **49".** Relatively pure copper methyl ester intermediate **51** (0.5048 g) was obtained from isocyanomethyl ester intermediate **41** (0.6590 g).
   Synthesis of copper salt methyl ester intermediate **52:** For specific operations, refer to "Synthesis of copper salt methyl ester intermediate **49".** Relatively pure copper salt methyl ester intermediate **52** (405.6 mg) was obtained from isocyanomethyl ester intermediate **42** (0.6418 g).
   Synthesis of copper salt methyl ester intermediate **53:** For specific operations, refer to "Synthesis of copper salt methyl ester intermediate **49".** Relatively pure copper salt methyl ester intermediate **53** (0.3732 g) was obtained from isocyanomethyl ester intermediate **43** (1.0229 g).
   Synthesis of copper salt methyl ester intermediate **54:** For specific operations, refer to "Synthesis of copper salt methyl ester intermediate **49".** Relatively pure copper salt methyl ester intermediate **54** (0.5547 g) was obtained from isocyanomethyl ester intermediate **44** (0.8140 g).
   Synthesis of copper salt methyl ester intermediate **55:** For specific operations, refer to "Synthesis of copper salt methyl ester intermediate **49".** Relatively pure copper salt methyl ester intermediate **55** (0.7437 g) was obtained from isocyanomethyl ester intermediate **45** (0.9372 g).
   Synthesis of copper salt methyl ester intermediate **56:** For specific operations, refer to "Synthesis of copper salt methyl ester intermediate **49".** Relatively pure copper salt methyl ester intermediate **56** (0.5368 g) was obtained from isocyanomethyl ester intermediate **46** (0.4487 g).
   Synthesis of copper salt methyl ester intermediate **57:** For specific operations, refer to "Synthesis of copper salt methyl ester intermediate **49".** Relatively pure copper salt methyl ester intermediate **57** (0.7204 g) was obtained from isocyanomethyl ester intermediate **47** (0.9000 g).
   Synthesis of copper salt methyl ester intermediate **58:** For specific operations, refer to "Synthesis of copper salt methyl ester intermediate **49".** Relatively pure copper salt methyl ester intermediate **58** (1.3991 g) was obtained from isocyanomethyl ester intermediate **48** (1.2494 g).
   Synthesis of copper salt carboxylic acid labeled precursor **59:** The copper salt methyl ester intermediate **49** (0.1560 g) and sodium hydroxide (0.0201 g) were stirred at room temperature for 5-6 h in a mixed solvent of tetrahydrofuran and water in a volume ratio of 4:1, acidified with dilute hydrochloric acid, extracted with dichloromethane, concentrated in vacuo, and separated by silica gel column chromatography to obtain relatively pure copper salt carboxylic acid labeled precursor **59** (0.0575 g). ¹H NMR (400 MHz, CD₃OD): δ 3.78 (s, 8H), 3.60 (t, *J =* 6.5 Hz, 8H), 2.69 (t, *J* = 6.5 Hz, 8H), 2.64 (t, *J =* 7.0 Hz, 8H), 2.20 (t, *J* = 7.0 Hz, 8H), 1.63-1.72 (m, 16H), 1.30 (s, 24H); ¹³C NMR (101 MHz, CD₃OD): δ 174.34, 162.55, 73.24, 62.14, 32.49, 32.37, 29.45, 25.46, 22.04; ¹⁹F NMR (376 MHz, CD₃OD): δ -155.44.
   Synthesis of copper carboxylate labeling precursor **60:** For specific operations, refer to "Synthesis of copper carboxylate labeling precursor **59".** Copper carboxylate labeling precursor **60** (0.1168 g) was obtained from copper methyl ester intermediate **50** (0.1799 g) and sodium hydroxide (0.0221 g). ¹H NMR (400 MHz, CD₃OD): δ 3.76 (s, 8H), 3.51 (t, *J =* 5.8 Hz, 8H), 2.64 (t, *J =* 7.2 Hz, 8H), 2.56 (t, *J* = 7.1 Hz, 8H), 2.21 (t, *J* = 7.2 Hz, 8H), 1.77-1.86 (m, 8H), 1.59-1.73 (m, 16H), 1.28 (s, 24H); ¹³C NMR (101 MHz, CD₃OD): δ 179.88, 157.70, 72.79, 59.89, 51.05, 36.33, 31.42, 29.90, 29.26, 28.49, 25.23, 22.09; ¹⁹F NMR (376 MHz, CD₃OD): δ -155.67.
   Synthesis of copper carboxylate labeling precursor **61:** For detailed operation, refer to "Synthesis of copper carboxylate labeling precursor **59".** Copper carboxylate labeling precursor **61** (0.2552 g) was obtained from copper methyl ester intermediate **51** (0.5048 g) and sodium hydroxide (0.0596 g). ¹H NMR (400 MHz, CD₃OD): δ 3.71 (s, 8H), 3.34 (t, *J* = 5.6 Hz, 8H), 2.48-2.57 (m, 16H), 2.11 (t, *J =* 7.2 Hz, 8H), 1.54-1.67 (m, 32H), 1.18 (s, 24H); ¹³C NMR (101 MHz, CD₃OD): δ 172.96, 157.45, 72.61, 61.40, 60.33, 50.85, 50.39, 36.63, 34.26, 32.77, 32.03, 31.79, 31.38, 29.36, 29.09, 28.90, 28.52, 26.48, 25.32, 25.23, 24.72, 22.30; ¹⁹F NMR (376 MHz, CD₃OD): δ -155.47; HRMS: calcd 350.0846 (M+Cu)⁺, found 350.0520 (M+Cu)⁺; calcd 637.2401 (2M+Cu)⁺, found 637.1989 (2M+Cu)⁺. When the compound is a copper salt, "M" in this paragraph refers to the exact mass of the isocyanate monomer in the copper salt, and the same applies below.
   Synthesis of copper salt carboxylic acid labeling precursor **62:** For specific operations, refer to "Synthesis of copper salt carboxylic acid labeling precursor **59".** Copper salt methyl ester intermediate **52** (0.1516 g) and sodium hydroxide (0.0596 g) were used to obtain copper salt carboxylic acid labeling precursor **62** (0.0967 g). ¹H NMR (400 MHz, CD₃OD): δ 3.76 (s, 8H), 3.43 (t, *J* = 5.6 Hz, 8H), 2.60 (q, *J* = 7.2 Hz, 16H), 2.21 (t, *J =* 6.8 Hz, 8H), 1.62-1.75 (m, 24H), 1.50-1.62 (m, 16H), 1.27 (s, 24H); ¹³C NMR (101 MHz, CD₃OD): δ 179.96, 146.11, 72.74, 61.24, 51.09, 36.36, 32.95, 32.93, 29.16, 28.97, 28.57, 25.74, 25.22, 22.01; ¹⁹F NMR (376 MHz, CD₃OD): δ - 155.47; HRMS: calcd 364.1002 (M+Cu)⁺, found 364.0928 (M+Cu)⁺; calcd 665.2714 (2M+Cu)⁺, found 665.2706 (2M+Cu)⁺.
   Synthesis of copper carboxylate labeling precursor **63:** For specific operations, refer to "Synthesis of copper carboxylate labeling precursor **59".** Copper carboxylate labeling precursor **63** (0.2106 g) was obtained from copper methyl ester intermediate **53** (0.3732 g) and sodium hydroxide (0.0407 g). HRMS: calcd 378.1159 (M+Cu)⁺, found 378.1063 (M+Cu)⁺; calcd 693.3027 (2M+Cu)⁺, found 693.3050 (2M+Cu)⁺.
   Synthesis of copper carboxylate labeling precursor **64:** For detailed operation, refer to "Synthesis of copper carboxylate labeling precursor **59".** Copper carboxylate labeling precursor **64** (0.1134 g) was obtained from copper methyl ester intermediate **54** (0.3017 g) and sodium hydroxide (0.0582 g). HRMS: calcd 392.1315 (M+Cu)⁺, found 392.1039 (M+Cu)⁺; calcd 721.3340 (2M+Cu)⁺, found 721.3295 (2M+Cu)⁺.
   Synthesis of copper carboxylate labeling precursor **65:** For detailed operation, refer to "Synthesis of copper carboxylate labeling precursor **59".** Copper carboxylate labeling precursor **65** (0.2530 g) was obtained from copper methyl ester intermediate **55** (0.7437 g) and sodium hydroxide (0.0680 g). HRMS: calcd 448.1941 (M+Cu)⁺, found 448.1981 (M+Cu)⁺.
   Synthesis of copper carboxylate labeling precursor **66:** For detailed operation, refer to "Synthesis of copper carboxylate labeling precursor **59".** Copper carboxylate labeling precursor **66** (0.2180 g) was obtained from copper methyl ester intermediate **56** (0.5368 g) and sodium hydroxide (0.0633 g). ¹H NMR (400 MHz, CD₃OD): δ 3.74 (s, 8H), 3.59 (t, *J =* 6.8 Hz, 8H), 2.67 (t, *J* = 6.8 Hz, 8H), 2.61 (t, *J* = 7.5 Hz, 8H), 2.16-2.27 (m, 8H), 1.56-1.64 (m, 16H), 1.39-1.45 (m, 8H), 1.33-1.38 (m, 8H), 1.28 (s, 24H).
   Synthesis of copper carboxylate labeling precursor **67:** For detailed operation, refer to "Synthesis of copper carboxylate labeling precursor **59".** Copper carboxylate labeling precursor **67** (0.0424 g) was obtained from copper methyl ester intermediate **57** (0.7204 g) and sodium hydroxide (0.0850 g). ¹H NMR (400 MHz, CD₃OD): δ 3.67 (s, 8H), 3.42 (t, *J =* 5.8 Hz, 8H), 2.55 (t, *J =* 7.2 Hz, 8H), 2.46 (t, *J =* 7.4 Hz, 8H), 2.10 (t, *J* = 7.5 Hz, 8H), 1.69-1.76 (m, 8H), 1.49-1.56 (m, 16H), 1.32-1.39 (m, 8H), 1.19 (s, 24H); ¹³C NMR (101 MHz, CD₃OD): δ 180.62, 157.70, 72.80, 59.87, 51.06, 36.97, 31.72, 29.92, 29.23, 28.65, 28.59, 25.69, 22.09; ¹⁹F NMR (376 MHz, CD₃OD): δ - 155.68.
   Synthesis of copper carboxylate labeling precursor **68:** For detailed operation, refer to "Synthesis of copper carboxylate labeling precursor **59".** Copper carboxylate labeling precursor **68** (0.4857 g) was obtained from copper methyl ester intermediate **58** (1.3991 g) and sodium hydroxide (0.1651 g). HRMS: calcd 350.0846 (M+Cu)⁺, found 350.0732 (M+Cu)⁺; calcd 637.2401 (2M+Cu)⁺, found 637.2467 (2M+Cu)⁺.
   Synthesis of Re (rhenium-186) complex **69:**
      (1) Preparation of freeze-dried kit: Dissolve tetra(2-methoxyisobutylisonitrile)copper(I) tetrafluoroborate (1.0 mg), copper salt carboxylic acid labeled precursor **59** (0.8 ~ 2.4 mg), stannous chloride dihydrate (0.025 ~ 0.075 mg), cysteine hydrochloride monohydrate (0.5 ~ 1.0 mg), sodium citrate dihydrate (1.0 ~ 2.6 mg) and D-mannitol (5 ~ 20 mg) in an appropriate amount of ultrapure water, adjust the pH to 5 ~ 6, and freeze-dry in a vial for later use.
      (2) Synthesis of Re complex: The above freeze-dried drug kit was dissolved in a mixed solvent of ethanol and water in a volume ratio of 1:1, and then ammonium perrhenate (2.5 ~ 3.9 mg) and stannous chloride dihydrate (2.1 ~ 3.3 mg) were added thereto, and the mixture was reacted at 100 °C for 30 min. After cooling, the mixture was filtered, and the filtrate was separated by HPLC (high pressure liquid chromatography) C-18 reverse phase semi-preparative column to obtain Re complex **69.** HRMS: calcd 1011.4997 (M)⁺, found 1011.5006 (M)⁺.
   Synthesis of Re complex **70:** For detailed procedures, refer to "Labeling of Re complex **69".** Re complex **70** was obtained by labeling precursor **60** (0.8 ~ 2.5 mg) with copper salt carboxylic acid.calcd 1025.5154 (M)⁺, found 1025.5131 (M)⁺. HRMS: calcd 1025.5154 (M)⁺, found 1025.5131 (M)⁺.
   Synthesis of Re complex **71:** For detailed procedures, refer to "Labeling of Re complex **69".** Re complex **71** was obtained by labeling precursor **61** (0.9 ~ 2.6 mg) with copper salt carboxylic acid. HRMS: calcd 1039.5283 (M)⁺, found 1039.5326 (M)⁺.
   Synthesis of Re complex **72:** For detailed procedures, refer to "Labeling of Re complex **69".** Re complex **72** was obtained by labeling precursor **62** (0.9 ~ 2.7 mg) with copper salt carboxylic acid. HRMS: calcd 1053.5439 (M)⁺, found 1053.5449 (M)⁺.
   Synthesis of Re complex **73:** For detailed procedures, refer to "Labeling of Re complex **69".** Re complex **73** was obtained by labeling precursor **63** (0.9 ~ 2.8 mg) with copper salt carboxylic acid. HRMS: calcd 1067.5629 (M)⁺, found 1067.5637 (M)⁺.
   Synthesis of Re complex **74:** For detailed procedures, refer to "Labeling of Re complex **69".** Re complex **74** was obtained by labeling precursor **64** (1.0 ~ 2.9 mg) with copper salt carboxylic acid. HRMS: calcd 1081.5752 (M)⁺, found 1081.5608 (M)^{+'.}
   Synthesis of Re complex **75:** For detailed procedures, refer to "Labeling of Re complex **69".** Re complex **75** was obtained by labeling precursor **66** (0.9 ~ 2.6 mg) with copper salt carboxylic acid. HRMS: calcd 1039.5283 (M)⁺, found 1039.4426 (M)⁺.
   Synthesis of Re complex **76:** For detailed procedures, refer to "Labeling of Re complex **69".** Re complex **76** was obtained by labeling precursor **67** (0.9 ~ 2.6 mg) with copper salt carboxylic acid. HRMS: calcd 1039.5283 (M)⁺, found 1039.4634 (M)⁺.
   Synthesis of Re complex **77:** For detailed procedures, refer to "Labeling of Re complex **69".** Re complex **77** was obtained by labeling precursor **68** (0.9 ~ 2.6 mg) with copper salt carboxylic acid. HRMS: calcd 1039.5283 (M)⁺, found 1039.4918 (M)⁺.
   Labeling of ^{99m}Tc (technetium-99m) complex **78:** In this embodiment, since the labeled ^{99m}Tc complex is radioactive, its mass spectrum cannot be directly measured in most cases. Therefore, the corresponding Re (rhenium-186) complex is usually synthesized and measured by HPLC C-18 reverse phase semi-preparative column. If there is no problem with the product, the ^{99m}Tc complex is prepared by the same method, and then the HPLC retention time of the Re complex and the corresponding ^{99m}Tc labeled product are compared to confirm the correctness of the chemical structure of the ^{99m}Tc labeled target product. This method is also used in other examples of the present invention.
      (1) Preparation of freeze-dried drug kit: Tetrakis (2-methoxyisobutylisonitrile) copper(I) tetrafluoroborate (1.0 mg), copper salt carboxylic acid labeled precursor **59** (0.8 ~ 2.4 mg), stannous chloride dihydrate (0.025 ~ 0.075 mg), cysteine hydrochloride monohydrate (0.5 ~ 1.0 mg), sodium citrate dihydrate (1.0 ~ 2.6 mg), and *D*-mannitol (5 ~ 20 mg) was dissolved in an appropriate amount of ultrapure water. And the pH was adjusted to 5 ~ 6, and lyophilized in a vial for later use.
      (2) ^{99m}Tc-Radiolabeling : The above lyophilized kit was dissolved in a mixed solvent of ethanol and water in a volume ratio of 1:1, and reacted with freshly washed Na^{99m}TcO₄ (37 ~ 3700 MBq) at 100 °C for 30 min. After cooling, the mixture was filtered and the filtrate was separated by HPLC C-18 reverse phase semi-preparative column to obtain ^{99m}Tc radioactive complex **78.** The HPLC spectrum of the co-injection of ^{99m}Tc radioactive complex **78** and the corresponding Re complex **69** (methanol/water containing 1‰ trifluoroacetic acid = 70/30, total flow rate 1.0 mL/min, C-18 reverse phase semi-preparative column) is shown in Fig. 1 (the blue fonts and curves in the Fig. represent the Re complex, and the red fonts and curves represent the ^{99m}Tc radioactive complex). The retention time of ^{99m}Tc radioactive complex **78** (61.531 min) is basically consistent with the retention time of the corresponding Re complex **69** (63.056 min).
   Labeling of ^{99m}Tc complex **79:** For specific operations, please refer to "Labeling of ^{99m}Tc complex **78".** ^{99m}Tc complex **79** was obtained by labeling precursor 61 (0.9 ~ 2.6 mg) with copper salt carboxylic acid. The HPLC spectrum of co-injection of ^{99m}Tc complex **79** and the corresponding Re complex **71** (methanol/water containing 1‰ trifluoroacetic acid = 70/30, total flow rate 1.0 mL/min, C-18 reverse phase semi-preparative column) is shown in Fig. 2. The retention time of ^{99m}Tc radioactive complex **79** (89.958 min) is basically consistent with the retention time of the corresponding Re complex **71** (91.575 min).
   Labeling of ^{99m}Tc complex **80:** For specific operations, see "Labeling of ^{99m}Tc complex **78".** The ^{99m}Tc radioactive complex 80 was obtained by labeling the precursor **62** (0.9 ~ 2.7 mg) with copper salt carboxylic acid. The HPLC spectrum of its co-injection with the corresponding Re complex **72** (methanol/water containing 1‰ trifluoroacetic acid = 73/27, total flow rate 1.0 mL/min, C-18 reverse phase semi-preparative column) is shown in Fig. 3. The retention time of the ^{99m}Tc radioactive complex **80** (80.675 min) is basically consistent with the retention time of the corresponding Re complex **72** (82.108 min).
   Labeling of ^{99m}Tc complex **81:** For specific operations, see "Labeling of ^{99m}Tc complex **78".** The ^{99m}Tc radioactive complex **81** was obtained by labeling the precursor **63** (0.9 ~ 2.8 mg) with copper salt carboxylic acid. The HPLC spectrum of its co-injection with the corresponding Re complex **73** (methanol/water containing 1‰ trifluoroacetic acid = 75.5/24.5, total flow rate 1.0 mL/min, C-18 reverse phase semi-preparative column) is shown in Fig. 4. The retention time of the 99mTc radioactive complex **81** (67.651 min) is basically consistent with the retention time of the corresponding Re complex **73** (69.084 min).
   Labeling of ^{99m}Tc complex **82:** For specific operations, see "Labeling of ^{99m}Tc complex **78".** The ^{99m}Tc radioactive complex **82** was obtained by labeling the precursor **64** (1.0 ~ 2.9 mg) with copper salt carboxylic acid. The HPLC spectrum of its co-injection with the corresponding Re complex **74** (methanol/water containing 1‰ trifluoroacetic acid = 78/22, total flow rate 1.0 mL/min, C-18 reverse phase semi-preparative column) is shown in Fig. 5. The retention time of the ^{99m}Tc radioactive complex **82** (70.350 min) was basically consistent with the retention time of the corresponding Re complex **74** (71.583 min).
   Labeling of ^{99m}Tc complex **83:** For specific operations, please refer to "Labeling of ^{99m}Tc complex **78".** The ^{99m}Tc radioactive complex **83** was obtained by labeling the precursor **66** (0.9 ~ 2.6 mg) with copper salt carboxylic acid. The liquid phase spectrum of co-injection of ^{99m}Tc radioactive complex **83** and the corresponding Re complex **76** (methanol/water containing 1‰ trifluoroacetic acid = 73/27, total flow rate 1.0 mL/min, C-18 reverse phase semi-preparative column) is shown in Fig. 6. The peak time of ^{99m}Tc radioactive complex **83** (73.083 min) was basically consistent with the retention time of the corresponding Re complex 75 (74.477 min).

### Example 2

The corresponding radioactive ^{99m}Tc complexes **78-81** of some typical compounds of the general formula **II** in this application were selected for in vivo biodistribution study in female Sprague-Dawley (SD) rats, as shown below:
(1) The experimental method is as follows:
Female SD rats (180-220 g, n = 3) were fasted for 12 h before the experiment, and the purified radioactive 99mTc complexes 78-81 were prepared into physiological saline (containing 10% ethanol) solutions of about 250-350 µCi/mL. 200 µL of the above solution was injected into the tail vein of female SD rats, and the rats were killed by cervical dislocation at five time points, 15, 30, 60, 120 and 240 min after the tail vein injection. The blood, brain, heart, liver, spleen, lung, kidney, muscle, bone, large intestine, small intestine, stomach and tail were collected, weighed and counted, and the count distribution of each tissue and organ was calculated (unit: %ID/g, the unit of large intestine, small intestine and stomach is %ID), and the data are the mean ± standard deviation of three mice in each phase. At the same time, 200 µL of the above solution was diluted to 20 mL, which was used as the %ID without deducting the tail count.
(2) The biodistribution results of radioactive ^{99m}Tc complex **78-81** in female SD rats are as follows:

**Table 1 Biodistribution data of radioactive ^{99m}Tc complex 79 in female SD rats (n = 3, ID%/g)**

| Organ/Tissue | Time (min) | | | | |
|---|---|---|---|---|---|
| | 15 | 30 | 60 | 120 | 240 |
| Blood | 0.11 ± 0.02 | 0.09 ± 0.01 | 0.04 ± 0.01 | 0.012 ± 0.002 | 0.025 ± 0.003 |
| Brain | 0.021 ± 0.005 | 0.012 ± 0.005 | 0.009 ± 0.001 | 0.009 ± 0.002 | 0.007 ± 0.002 |
| Heart | 1.50 ± 0.27 | 2.24 ± 0.31 | 2.45 ± 0.35 | 1.54 ± 0.45 | 0.60 ± 0.04 |
| Liver | 1.28 ± 0.19 | 1.38 ± 0.16 | 0.66 ± 0.12 | 0.28 ± 0.01 | 0.24 ± 0.01 |
| Spleen | 0.52 ± 0.10 | 0.16 ± 0.01 | 0.09 ± 0.02 | 0.05 ± 0.01 | 0.11 ± 0.01 |
| Lungs | 0.30 ± 0.06 | 0.27 ± 0.03 | 0.14 ± 0.02 | 0.11 ± 0.03 | 0.09 ± 0.01 |
| Kidney | 4.87 ± 0.72 | 3.37 ± 0.38 | 1.65 ± 0.53 | 0.65 ± 0.14 | 0.22 ± 0.02 |
| Muscle | 0.24 ± 0.06 | 0.81 ± 0.14 | 0.47 ± 0.10 | 0.21 ± 0.05 | 0.27 ± 0.02 |
| Bone | 0.13 ± 0.02 | 0.09 ± 0.01 | 0.05 ± 0.01 | 0.04 ± 0.01 | 0.13 ± 0.03 |
| Large Intestine | 0.76 ± 0.20 | 12.06 ± 3.78 | 16.70 ± 2.72 | 27.61 ± 2.20 | 33.40 ± 4.10 |
| Small Intestine | 14.70 ± 0.78 | 25.98 ± 3.13 | 22.54 ± 5.67 | 5.24 ± 0.78 | 4.53 ± 0.88 |
| Stomach | 0.51 ± 0.05 | 0.47 ± 0.15 | 0.19 ± 0.06 | 0.22 ± 0.03 | 0.20 ± 0.02 |
| Heart-to-Blood | 13.99 ± 3.55 | 25.88 ± 5.10 | 56.19 ± 13.56 | 118.25 ± 44.79 | 24.36 ± 1.72 |
| Heart-to-Liver | 1.22 ± 0.33 | 1.63 ± 0.27 | 3.82 ± 1.21 | 5.55 ± 1.38 | 2.45 ± 0.18 |
| Heart-to-Lungs | 5.16 ± 0.69 | 8.21 ± 0.51 | 17.12 ± 3.09 | 15.05 ± 5.27 | 6.66 ± 0.60 |

In terms of absolute myocardial uptake values, the absolute myocardial uptake values of our radioactive ^{99m}Tc complex 79 in female SD rats were 1.50 ± 0.27, 2.24 ± 0.31, 2.45 ± 0.35, 1.54 ± 0.45 and 0.60 ± 0.04 %ID/g at 15, 30, 60, 120 and 240 min post-injection, respectively; among them, the absolute myocardial uptake reached a peak value of 2.45 ± 0.35 %ID/g at 60 min post-injection; although this peak value was different from the absolute myocardial uptake peak values of ^{99m}Tc-MIBI (the most widely used myocardial perfusion imaging agent in clinical practice in the world) and [¹²³I]BMIPP (the only fatty acid imaging agent approved for clinical use in the world) in rats, this absolute myocardial uptake value was good and acceptable. In addition, our radiotracer showed a long myocardial retention time within 15-240 min post-injection, but also showed an obvious dynamic trend of first increasing and then decreasing, while the absolute myocardial uptake value of ^{99m}Tc-MIBI changed little with the extension of time after injection. Therefore, compared with ^{99m}Tc-MIBI, our radioactive ^{99m}Tc complex 79 can better reflect the strength of myocardial vitality and metabolic function.

According to literature records, the absolute myocardial uptake values of the myocardial perfusion imaging agent ^{99m}Tc-MIBI in female SD rats were 3.70, 3.16 and 3.04%Dose/g at 10, 30 and 60 min post-injection, respectively, see reference 1; 3.26 ± 0.18, 3.07 ± 0.21, 3.13 ± 0.12 and 3.29 ± 0.12%ID/g at 10, 20, 30 and 60 min after injection, respectively, see reference 2; 3.16 ± 0.56, 3.14 ± 0.42 and 2.83 ± 0.25%ID/g at 30, 60 and 120 min after injection, respectively, see reference 3. The absolute myocardial uptake values of the fatty acid imaging agent [¹²³I]BMIPP in rats were 3.63, 4.26 and *2.27%ID*/*g* at 30, 60 and 240 min post-injection, respectively, see reference 4.

Within 15-120 min post-injection, the uptakes in liver and lung of our radioactive ^{99m}Tc complex 79 in female SD rats gradually decreased, and the heart-to-liver ratios gradually increased. Especially within 60-120 min post-injection, the absolute uptake value of myocardium in female SD rats significantly exceeded the liver background.

The heart-to-liver ratios of our radioactive ^{99m}Tc complex 79 were 3.82 ± 1.21 and 5.55 ± 1.38 at 60 and 120 min post-injection in female SD rats, respectively. The heart-to-liver ratios were high, exceeding the corresponding values of ^{99m}Tc-MIBI at the same time point in female SD rats and the corresponding value of [¹²³I]BMIPP at 60 min post-injection in rats.

According to the literature, the heart-to-liver ratios of the myocardial perfusion imaging agent ^{99m}Tc-MIBI in female SD rats are: 2.04 ± 0.17 and 2.96 ± 0.34 at 60 and 120 min post-injection, respectively, see reference 1; 2.65 ± 0.22 at 60 min after injection, see reference 2; 4.25 ± 0.84, and 4.52 ± 1.57 at 60 and 120 min after injection, see reference 3. The heart-to-liver ratios of the fatty acid imaging agent [¹²³I]BMIPP in rats are: 2.22 and 2.39 at 60 and 240 min after injection, respectively, see reference 4.

At 30, 60, 120 and 240 min after intravenous injection, the heart-to-lungs ratios of our radioactive ^{99m}Tc complex 79 were 8.21 ± 0.51, 17.12 ± 3.09, 15.05 ± 5.27 and 6.66 ± 0.60, respectively. The heart-to-lungs ratios were very high, exceeding the corresponding values of ^{99m}Tc-MIBI at 30, 60 and 120 min post-injection in female SD rats, and also far exceeding the corresponding values of [¹²³I]BMIPP at 30, 60 and 240 min post-injection in rats.

The heart-to-lungs ratios of the myocardial perfusion imaging agent ^{99m}Tc-MIBI in female SD rats were 5.17 ± 0.39, 6.77 ± 0.55 and 6.48 ± 0.51 at 30, 60 and 120 min post-injection, respectively, see reference 1; 4.66 ± 0.24 and 7.32 ± 0.28 at 30 and 60 min post-injection, respectively, see reference 2; 3.57 ± 0.72, 6.41 ± 1.25, and 7.97 ± 1.89 at 30, 60 and 120 min post-injection, respectively, see reference 3. The heart-to-lungs ratios of the fatty acid imaging agent [¹²³I]BMIPP in rats were 3.16, 3.52 and 2.49 at 30, 60 and 240 min after injection, respectively, see reference 4.

The heart-to-blood ratios of our radioactive ^{99m}Tc complex 79 were very high. At 30, 60, 120 and 240 min post-injection, its heart-to-blood ratios were 25.88 ± 5.10, 56.19 ± 13.56, 118.25 ± 44.79, and 24.36 ± 1.72, respectively, which far exceeds the corresponding values of [¹²³I]BMIPP at 30, 60 and 240 min post-injection in rats. Although the heart-to-blood ratios of our radiopharmaceutical were lower than the corresponding values of ^{99m}Tc-MIBI at 30 and 60 min post-injection in female SD rats, its heart-to-blood ratios were still very high. This was because when the heart-to-blood ratios were greater than 10, the difference in heart/blood value had almost negligible effect on myocardial imaging.

According to the literature, the heart-to-blood ratios of the myocardial perfusion imaging agent ^{99m}Tc-MIBI in female SD rats were 104.30 and 164.50 at 30 and 60 min post-injection, respectively, see reference 2; and 114.6 ± 34.70, 137.80 ± 35.00 and 209.00 ± 12.40 at 30, 60 and 120 min after injection, respectively, see reference 3. The heart-to-blood ratios of the fatty acid imaging agent [¹²³I]BMIPP in rats were 2.27, 2.51 and 2.01 at 30, 60 and 240 min post-injection, respectively, see reference 4.

The above-mentioned reference 1 is: Boschi, A.; Uccelli, L.; Bolzati, C.; Duatti, A.; Sabba, N.; Moretti, E.; Di Domenico, G.; Zavattini, G.; Refosco, F.; Giganti, M. Synthesis and Biologic Evaluation of Monocationic Asymmetric 99mTc-Nitride Heterocomplexes Showing High Heart Uptake and Improved Imaging Properties. J. Nucl. Med. 2003, 44 (5), 806-814. The above-mentioned reference 2 is: Hatada, K.; Riou, L. M.; Ruiz, M.; Yamamichi, Y.; Duatti, A.; Lima, R. L.; Goode, A. R.; Watson, D. D.; Beller, G. A.; Glover, D. K. 99mTc-N-DBODC5, a New Myocardial Perfusion Imaging Agent with Rapid Liver Clearance: Comparison with 99mTc-Sestamibi and 99™Tc-Tetrofosmin in Rats. J. Nucl. Med. 2004, 45 (12), 2095-2101. The above-mentioned reference3 is: Liu, S.; He, Z.-J.; Hsieh, W.-Y.; Kim, Y.-S. Evaluation of Novel Cationic 99mTc-Nitrido Complexes as Radiopharmaceuticals for Heart Imaging: Improving Liver Clearance with Crown Ether Groups. Nucl. Med.Bio. 2006, 33 (3), 419-432. The above-mentioned reference 4 is: Goodman, M. M.; Kirsch, G.; Knapp Jr., F. F. Synthesis and Evaluation of Radioiodinated Terminal p-Iodophenyl-Substituted α- and β-Methyl-branched Fatty Acids. J. Med. Chem. 1984, 27 (3), 390-397.

**Table 2 Biodistribution data of radioactive ^{99m}Tc complex 80 in female SD rats (n = 3, ID%/g)**

| Organ/Tis sue | Time (min) | | | | | |
|---|---|---|---|---|---|---|
| | 15 | 30 | 60 | 120 | 240 | 60 (blocked by trimetazidine hydrochloride) |
| Blood | 0.018 ± 0.003 | 0.026 ± 0.003 | 0.013 ± 0.003 | 0.009 ± 0.001 | 0.007 ± 0.001 | 0.024 ± 0.003 |
| Brain | 0.008 ± 0.004 | 0.010 ± 0.003 | 0.011 ± 0.005 | 0.010 ± 0.005 | 0.007 ± 0.002 | 0.009 ± 0.005 |
| Heart | 0.94 ± 0.16 | 1.42 ± 0.32 | 2.06 ± 0.06 | 1.73 ± 0.09 | 0.93 ± 0.09 | 1.01 ± 0.19 |
| Liver | 0.49 ± 0.03 | 1.01 ± 0.29 | 0.33 ± 0.09 | 0.18 ± 0.01 | 0.17 ± 0.03 | 0.51 ± 0.16 |
| Spleen | 0.23 ± 0.06 | 0.26 ± 0.06 | 0.16 ± 0.06 | 0.15 ± 0.01 | 0.06 ± 0.01 | 0.10 ± 0.04 |
| Lungs | 0.14 ± 0.04 | 0.32 ± 0.04 | 0.22 ± 0.03 | 0.16 ± 0.01 | 0.06 ± 0.01 | 0.16 ± 0.02 |
| Kidney | 2.43 ± 0.10 | 4.66 ± 0.51 | 2.39 ± 0.58 | 1.30 ± 0.41 | 0.49 ± 0.03 | 0.97 ± 0.28 |
| Muscle | 0.14 ± 0.04 | 0.22 ± 0.07 | 0.48 ± 0.06 | 0.34 ± 0.03 | 0.22 ± 0.02 | 0.31 ± 0.06 |
| Bone | 0.11 ± 0.05 | 0.10 ± 0.01 | 0.064 ± 0.004 | 0.07 ± 0.02 | 0.043 ± 0.003 | 0.07 ± 0.01 |
| Large Intestine | 5.34 ± 1.37 | 6.69 ± 0.68 | 12.36 ± 2.55 | 22.13 ± 2.94 | 22.41 ± 1.13 | 15.54 ± 6.05 |
| Small Intestine | 8.49 ± 0.85 | 10.71 ± 1.89 | 6.75 ± 1.89 | 5.02 ± 0.82 | 7.36 ± 0.64 | 16.59 ± 4.32 |
| Stomach | 0.27 ± 0.09 | 0.53 ± 0.07 | 0.56 ± 0.27 | 0.55 ± 0.08 | 0.20 ± 0.02 | 1.10 ± 0.19 |
| Heart-to-Blood | 53.37 ± 6.93 | 54.80 ± 11.86 | 164.01 ± 43.51 | 197.36 ± 32.29 | 141.90 ± 22.86 | 41.25 ± 3.12 |
| Heart-to-Liver | 1.91 ± 0.40 | 1.48 ± 0.45 | 6.43 ± 1.85 | 9.68 ± 0.76 | 5.56 ± 1.30 | 2.06 ± 0.41 |
| Heart-to-Lungs | 7.04 ± 1.69 | 4.40 ± 0.64 | 9.48 ± 1.39 | 11.40 ± 3.06 | 15.00 ± 3.18 | 6.28 ± 0.29 |

For the absolute myocardial uptake values, heart-to-liver ratios, heart-to-lungs ratios, and heart-to-blood ratios of the myocardial perfusion imaging agent ^{99m}Tc-MIBI in female SD rats, and the corresponding values of the fatty acid imaging agent [¹²³I]BMIPP in rats, please refer to the relevant part of the discussion on the biodistribution results of the radioactive ^{99m}Tc complex 79 after Table 1 in this example.

In terms of absolute myocardial uptake values, the absolute myocardial uptake values of our radioactive ^{99m}Tc complex 80 in female SD rats were 0.94 ± 0.16, 1.42 ± 0.32, 2.06 ± 0.06, 1.73 ± 0.09, and 0.93 ± 0.09 %ID/g at 15, 30, 60, 120 and 240 min post-injection, respectively; among them, the absolute myocardial uptake reached a peak value of 2.06 ± 0.06 %ID/g at 60 min post-injection; although this peak value was different from the absolute myocardial uptake peak values of ^{99m}Tc-MIBI (the most widely used myocardial perfusion imaging agent in clinical practice in the world) and [¹²³I]BMIPP (the only fatty acid imaging agent approved for clinical use in the world) in rats, this absolute myocardial uptake value is also acceptable. Moreover, ^{99m}Tc complex **80,** like the above-mentioned ^{99m}Tc complex **79,** also retains in the myocardium for a long time within 15-240 min post-injection, but also shows an obvious dynamic trend of first increasing and then decreasing (among which, the absolute myocardial uptake value of ^{99m}Tc complex **80** decreased by 54.9% within 60-240 min post-injection, which was a significant decrease). However, the absolute myocardial uptake value of ^{99m}Tc-MIBI changed little with the extension of time after injection. Therefore, compared with ^{99m}Tc-MIBI, our radioactive ^{99m}Tc complex **80** can better reflect the strength of myocardial vitality and metabolic function.

Within 30-120 min post-injection, the uptakes in liver and lungs of our radioactive ^{99m}Tc complex **80** in female SD rats gradually decreased, and the heart-to-liver ratios gradually increased. In particular, within 60-120 min post- injection, the absolute uptake value of myocardium in female SD rats also significantly exceeded the liver background.

The heart-to-liver ratios of ^{99m}Tc complex **80** at 60, 120 and 240 min post-injection in female SD rats were 6.43 ± 1.85, 9.68 ± 0.76 and 5.56 ± 1.30, respectively. The heart-to-liver ratios were high, significantly exceeding the corresponding values of ^{99m}Tc-MIBI at the same time post-injection in female SD rats, and the corresponding values of [¹²³I]BMIPP at 60 and 240 min post-injection in rats.

At 60, 120 and 240 min post-injection, the heart-to-lungs ratios of ^{99m}Tc complex **80** were 9.48 ± 1.39, 11.40 ± 3.06 and 15.00 ± 3.18, respectively. The heart-to-lungs ratios were very high, significantly exceeding the corresponding values of ^{99m}Tc-MIBI at 30, 60 and 120 min post-injection in female SD rats, and also far exceeding the corresponding values of [¹²³I]BMIPP at 30, 60 and 240 min post-injection in rats.

The heart-to-blood ratios of radioactive ^{99m}Tc complex **80** were also very high, with heart-to-blood ratios of 54.80 ± 11.86, 164.01 ± 43.51, 197.36 ± 32.29, and 141.90 ± 22.86 at 30, 60, 120, and 240 min post-injection, respectively, which were far higher than the corresponding values of [¹²³I]BMIPP at 30, 60 and 240 min post-injection in rats. The heart-to-blood ratios of this ^{99m}Tc complex were also close to the corresponding values of ^{99m}Tc-MIBI (104.30 and 164.50 at 30 and 60 min post-injection) in female SD rats, indicating that its heart-to-blood ratios were still very high. This was because when the heart-to-blood ratios were greater than 10, the difference in heart-to-blood ratios had almost negligible effect on myocardial imaging.

Trimetazidine hydrochloride (TMZ) is a widely used anti-anginal drug and an inhibitor of fatty acid β-oxidation. TMZ directly inhibits fatty acid oxidation (FAO) in the β-oxidation pathway because it strongly inhibits long-chain 3-ketoacyl-CoA (CoA) sulfidases (enzymes that catalyze the last step of fatty acid β-oxidation). It can also inhibit medium-chain or short-chain 3-ketoacyl-CoA sulfidases to a certain extent. Therefore, in order to investigate the sensitivity of radioactive ^{99m}Tc complex **80** to fatty acid β-oxidation, we first performed a TMZ blocking experiment from the perspective of biodistribution. Among them, SD rats killed 60 min after injection were divided into two groups: one group of fasting female SD rats were injected with saline (0.2 mL) solution containing TMZ (40 mg/kg) and our radiotracer ^{99m}Tc complex **80** (TMZ administration group), and the other group of fasting female SD rats were injected with radioactive ^{99m}Tc complex **80** only (control group). The absolute myocardial uptake of the control SD rats and the TMZ-administered SD rats was compared 60 min after the tail vein injection of our radiotracer **80** (corresponding to the time point of the peak absolute myocardial uptake). As can be seen from Table 2, at 60 min post-injection, the absolute uptake value of ^{99m}Tc complex **80** in the TMZ-administered SD rats was significantly reduced compared with the corresponding value in the control SD rats, with a decrease of 51% (significant P value <0.01). This indicates that the radioactive ^{99m}Tc complex **80** is quite sensitive to myocardial fatty acid β-oxidation. The effect of TMZ administration on the metabolic fate of the radioactive ^{99m}Tc complex **80** will also be discussed in Example 4 below.

**Table 3 Biodistribution data of radioactive ^{99m}Tc complex 81 in female SD rats (n = 3, ID%/g)**

| Organ/Tissue | Time (min) | | | | |
|---|---|---|---|---|---|
| | 15 | 30 | 60 | 120 | 240 |
| Blood | 0.018 ± 0.001 | 0.016 ± 0.002 | 0.009 ± 0.001 | 0.020 ± 0.004 | 0.016 ± 0.002 |
| Heart | 3.37 ± 0.15 | 3.45 ± 0.19 | 3.98 ± 0.25 | 2.83 ± 0.25 | 2.29 ± 0.05 |
| Liver | 1.79 ± 0.26 | 1.11 ± 0.16 | 0.60 ± 0.12 | 0.36 ± 0.05 | 0.17 ± 0.03 |
| Spleen | 0.70 ± 0.06 | 0.88 ± 0.13 | 0.34 ± 0.04 | 0.25 ± 0.04 | 0.12 ± 0.02 |
| Lungs | 0.86 ± 0.17 | 0.575 ± 0.002 | 0.28 ± 0.07 | 0.27 ± 0.03 | 0.15 ± 0.02 |
| Kidney | 9.30 ± 0.20 | 5.21 ± 1.54 | 4.90 ± 0.17 | 4.03 ± 0.24 | 2.07 ± 0.17 |
| Muscle | 0.72 ± 0.11 | 0.37 ± 0.06 | 0.77 ± 0.13 | 0.60 ± 0.11 | 0.48 ± 0.06 |
| Bone | 0.24 ± 0.05 | 0.15 ± 0.01 | 0.14 ± 0.01 | 0.12 ± 0.01 | 0.036 ± 0.005 |
| Large Intestine | 6.53 ± 0.73 | 14.01 ± 1.87 | 6.44 ± 0.70 | 19.30 ± 1.11 | 27.81 ± 4.20 |
| Small Intestine | 12.23 ± 2.35 | 17.82 ± 0.19 | 15.98 ± 1.55 | 24.46 ± 3.26 | 13.00 ± 1.55 |
| Stomach | 1.05 ± 0.04 | 0.65 ± 0.11 | 0.46 ± 0.11 | 0.92 ± 0.03 | 0.30 ± 0.08 |
| Heart-to-Blood | 192.24 ± 13.25 | 220.61 ± 45.73 | 449.52 ± 67.26 | 146.14 ± 22.27 | 143.42 ± 25.82 |
| Heart-to-Liver | 1.93 ± 0.26 | 3.30 ± 0.42 | 6.87 ± 1.56 | 7.91 ± 0.50 | 11.82 ± 1.15 |
| Heart-to-Lungs | 4.44 ± 0.26 | 6.00 ± 0.31 | 14.35 ± 2.38 | 10.43 ± 0.62 | 15.97 ± 2.41 |

For the absolute myocardial uptake values, heart-to-liver ratios, heart-to-lungs ratios, and hear-to-blood ratios of the myocardial perfusion imaging agent ^{99m}Tc-MIBI in female SD rats, and the corresponding values of the fatty acid imaging agent [¹²³I]BMIPP in rats, please refer to the relevant part of the discussion on the biodistribution results of the radioactive ^{99m}Tc complex 79 after Table 1 in this example.

The absolute myocardial uptake values of radioactive ^{99m}Tc complex 81 in female SD rats were 3.37 ± 0.15, 3.45 ± 0.19, 3.98 ± 0.25, 2.83 ± 0.25 and 2.29 ± 0.05%ID/g at 15, 30, 60, 120 and 240 min post-injection, respectively. The myocardial uptake values were generally high. Among them, the absolute myocardial uptake reached a high peak of 3.98 ± 0.25 %ID/g at 60 min post-injection. This peak value was close to the absolute myocardial uptake peak values of ^{99m}Tc-MIBI (the most widely used myocardial perfusion imaging agent in clinical practice in the world) and [¹²³I]BMIPP (the only fatty acid imaging agent approved for clinical use in the world) in rats. In addition, our radioactive drug has a long myocardial retention time of 15-240 min post-injection, but also shows a relatively obvious dynamic trend of first increasing and then decreasing, while the absolute myocardial uptake value of ^{99m}Tc-MIBI changes little with the extension of time after injection. Therefore, compared with 99mTc-MIBI, our radioactive ^{99m}Tc complex **81** could better reflect the strength of myocardial vitality and metabolic function.

In the 15-240 min post-injection, the radioactive ^{99m}Tc complex **81** also gradually decreased the liver and lung background in female SD rats, similar to complex **79,** and gradually increased the heart-to-liver ratios. In particular, in the 60-240 min post-injection, the absolute myocardial uptakes in female SD rats significantly exceeded the liver and lung uptakes.

The heart-to-liver ratios of the radioactive ^{99m}Tc complex **81** at 60, 120 and 240 min post-injection in female SD rats were 6.87 ± 1.56, 7.91 ± 0.50 and 11.82 ± 1.15, respectively. The heart-to-liver uptakes were high, significantly exceeding the corresponding values of ^{99m}Tc-MIBI at the same time points after tail vein injection in female SD rats, and the corresponding values of [¹²³I]BMIPP at 60 and 240 min post-injection in rats.

At 30, 60, 120 and 240 min post-injection, the heart-to-liver ratios of our radioactive ^{99m}Tc complex **81** were 6.00 ± 0.31, 14.35 ± 2.38, 10.43 ± 0.62 and 15.97 ± 2.41, respectively. The heart-to-lungs ratios were very high, significantly exceeding the corresponding values of ^{99m}Tc-MIBI at 30, 60 and 120 min post-injection in female SD rats, and also far exceeding the corresponding values of [¹²³I]BMIPP at 30, 60 and 240 min post-injection in rats.

The heart-to-blood ratios of our radioactive ^{99m}Tc complex **81** was very high. At 30, 60, 120 and 240 min post-injection, its heart-to-blood ratios were 220.61 ± 45.73, 449.52 ± 67.26, 146.14 ± 22.27, and 143.42 ± 25.82, respectively, which far exceeded the corresponding values of [¹²³I]BMIPP at 30, 60 and 240 min post-injection in rats. Although the heart-to-blood value of our radiopharmaceutical was lower than the corresponding values of ^{99m}Tc-MIBI at 30 and 60 min post-injection in female SD rats, its heart-to-blood ratios were still very high. This was because when the heart-to-blood ratios were greater than 10, the difference in heart-to-blood ratios had almost negligible effect on myocardial imaging.

The absolute myocardial uptake values and heart-to-liver ratios of radioactive ^{99m}Tc complex **78** were lower than the above values.

In summary, it could be seen from the test result of the present embodiment that among radioactive ^{99m}Tc complexes **78-81,** along with the growth of fatty acid carbon chain length, its myocardial absolute uptake peak value in female SD rats is generally on the rise. Especially for ^{99m}Tc complex **81,** myocardial absolute uptake peak value was close to the corresponding value of myocardial perfusion imaging agent ^{99m}Tc-MIBI, which is currently the most widely used in clinical application in the world. The myocardial absolute uptake peak value of ^{99m}Tc complexes **79-80** was also good. The myocardial uptake value of ^{99m}Tc complexes **79-81** gradually increased during 15-60 min post-injection, and then decreased during 60-240 min post-injection, This showed the dynamic change of myocardial absorption. And, the overall biodistribution results of the above 3 complexes were excellent.

### Example 3

The corresponding radioactive ^{99m}Tc complexes **79-80** of some typical compounds of general formula **II** in this application were selected for small animal SPECT/CT imaging study of female SD rats, as shown below:
(1) The experimental method is as follows:
   Female SD rats (180 g, fasted overnight) were anesthetized with 2% isoflurane gas and initially injected with 800 µCi of radioactive ^{99m}Tc complex **39-40** (dissolved in saline containing 10% EtOH, 1000 µL) via the tail vein, and then SPECT/CT scanning was performed.
(2) Experimental results:
   Fig.7 corresponds to representative dynamic SPECT/CT images of radioactive ^{99m}Tc complex **79** in female SD rats (A, B and C of Fig. 7 were coronal, sagittal, and cross-sectional images of dynamic SPECT/CT images of ^{99m}Tc complex **79** at 56-64 min post-injection of female SD rats, respectively);
   Fig. 8 corresponds to representative dynamic SPECT/CT images of radioactive ^{99m}Tc complex **80** in female SD rats (A, B and C of Fig. 8 are coronal, sagittal, and cross-sectional images of dynamic SPECT/CT images of ^{99m}Tc complex **80** at 66-72 min post-injection of female SD rats, respectively);
   As can be seen from Figs. 7 and 8, at 56-64 min post-injection of SD rats, the radioactive ^{99m}Tc complex **79,** and at 66-72 min post-injection of SD rats, the two radioactive ^{99m}Tc complexes were imaged very clearly in the myocardium, while the lung, blood and liver background were almost unobservable at this time, indicating that the myocardial imaging quality of the two radioactive ^{99m}Tc complexes was excellent.

### Example 4

The corresponding radioactive ^{99m}Tc complex **80** of some typical compounds of general formula **II** in this application was selected for metabolism study in female SD rats, as shown below:
(1) Experimental method

Female SD rats (180-220 g, fasted overnight) were injected with 800 µCi of radioactive ^{99m}Tc complex **80** (200-300 µCi, dissolved in 10% EtOH saline, 1000 µL) (n = 3) through the tail vein. Heart, intestine and liver samples were collected at 60 min and 120 min after injection. Among them, the SD rats killed at 60 min post-injection were divided into two groups: one group of fasting female SD rats were injected with saline (0.2 mL) solution containing fatty acid β-oxidation inhibitor TMZ (40 mg/kg) and our radiotracer (TMZ-administratied group), and the other group of fasting female SD rats were injected with radioactive ^{99m}Tc complex 80 only (control group). Heart, intestine or liver samples were homogenized in a mixture of CHCl₃-CH₃OH in a volume ratio of 2:1, then 40% urea and 5% sulfuric acid aqueous solution were added, and they were sonicated and centrifuged at 2000 rpm for 10 min (a modified Folch extraction method). The ^{99m}Tc gamma counting were performed on the resulting aqueous phase, organic phase and residual tissue particle phase (n = 3).

In addition, the residual tissue particles obtained from the heart samples were measured for radioactivity counts, then heated and dissolved in 1 mol/L NaOH solution at 90 °C for 1 h, cooled, and then treated with 50% trichloroacetic acid (TCA). After centrifugation at 2000 rpm for 10 min, the obtained supernatant and precipitate were ^{99m}Tc gamma counting.

### (2) Experimental results

Table 4 Distribution of ^{99m}Tc radioactivity in the aqueous phase, organic phase, and residual tissue particle phase of the heart, liver, and intestine of fasted female SD rats after tissue extraction by the modified Folch extraction method (percentage of total ^{99m}Tc radioactivity counts) (60 and 120 min post-injection of ^{99m}Tc complex **80,** n = 3)

**Table 4 Metabolism of radioactive 99mTc complex 80 in female SD rats**

| Organ/Tissue | Time | %organic fraction | %aqueous fraction | %residual tissue pellet |
|---|---|---|---|---|
| | | ^{99m}Tc complex **80** | | |
| heart in control SD rats (n = 3) | 60 min | 31.38 ± 2.48 | 0.50 ± 0.21 | 68.12 ± 2.69 |
| | 120 min | 33.20 ± 4.56 | 0.53 ± 0.23 | 66.27 ± 4.54 |
| heart in TMZ-administrated SD rats (n = 3) | 60 min | 73.11 ± 2.03 | 0.29 ± 0.06 | 26.60 ± 2.09 |
| liver in control SD rats (n = 3) | 60 min | 35.30 ± 4.75 | 0.66 ± 0.21 | 64.04 ± 4.90 |
| | 120 min | 31.92 ± 3.83 | 0.85 ± 0.30 | 67.23 ± 4.08 |
| intestine in control SD rats (n = 3) | 60 min | 38.76 ± 5.69 | 0.54 ± 0.24 | 60.70 ± 5.71 |
| | 120 min | 32.77 ± 3.98 | 1.68 ± 0.44 | 65.55 ± 3.54 |

The results of the modified Folch extraction analysis of tissue extracts showed that most of the ^{99m}Tc radioactivity in the heart, intestine, and liver was present in the residual tissue particulate phase at 60 and 120 min after intravenous injection of ^{99m}Tc complex **80,** as shown in Table 4. The proportion of ^{99m}Tc radioactivity counts in the residual tissue particulate phase in the heart samples of the control SD rats at these two time points was 68.54 ± 3.66% and 66.27 ± 34.54% of the total counts, respectively, in the liver samples, the corresponding values were 64.04 ± 4.90% and 67.23 ± 34.08%, and in the intestinal samples, the corresponding values were 60.70 ± 5.71% and 65.55 ± 3.54%, respectively. In addition, for the heart samples of the control SD rats at 60 min after the ^{99m}Tc complex **80,** when the residual tissue granular phase was dissolved in 1 mol/L NaOH aqueous solution at 90 °C for 1 h, most of the ^{99m}Tc radioactivity in the residual tissue granular phase was precipitated again when 50% trichloroacetic acid was added (the precipitated ^{99m}Tc radioactivity count accounted for 86.0% of the ^{99m}Tc radioactivity count in the residual tissue granular phase). In addition, compared with the SD rats in the control group, the proportion of ^{99m}Tc radioactivity counts in the residual tissue granular phase (the percentage of the total radioactivity counts) was significantly reduced from 68.54 ± 3.66% (control SD rat heart samples) to 26.60 ± 2.09% (TMZ-treated SD rat heart samples) at 60 min after the injection of the ^{99m}Tc complex **80,** and the value was reduced by 61% (significant P value <0.01). At 60 min post-injection of ^{99m}Tc complex **80,** the content of the organic part of the heart sample increased from 31.38 ± 2.48% (heart sample of SD rats in the control group) to 73.11 ± 2.03% (heart sample of SD rats in the TMZ administration group), and the significant P value was <0.01 compared with the control group SD rats. The results of the above metabolic analysis, combined with the results of the TMZ inhibition test from the perspective of biodistribution in Example 2, show that the sensitivity of radioactive ^{99m}Tc complex **80** to myocardial fatty acid β-oxidation is quite high. Moreover, most of the ^{99m}Tc complex 80 was partially β-oxidized to radioactive metabolites that can bind tightly to tissue proteins, which provides strong evidence for the β-oxidation of our radiotracer in the myocardium.

It could be clearly seen from Examples 2, 3, and 4 that the present application adds a compound with thio added at J, and compared with the case without thio, the uptakes in the lungs and blood were greatly reduced; the uptakes in the liver was cleared very quickly, while the myocardial imaging was very obvious. And the water solubility was enhanced, which were not found in previous studies of fatty acids. The metabolic characteristics of the structure in the present application were very similar to those of an ideal imaging agent, and it fully embodied the characteristics of a myocardial metabolic imaging agent. The imaging agent of the present invention could better reflect the vitality and metabolic state of the myocardium, and could be better used in the diagnosis of heart disease and the judgment of myocardial cell survival. This allowed it to be used as a myocardial fatty acid metabolic imaging agent, and had important clinical application prospects.

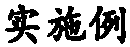 **5**

The organic synthesis routes and methods of the labeled precursors **96-98** of the typical compounds of the general formula III in this application, as well as the labeling routes and methods of the Re (thenium-186) complex **99** of some typical compounds (when f in the general formula III is 5, 7, 11, and g is 4) were selected as follows:
(1) The synthesis and labeling routes are as follows: 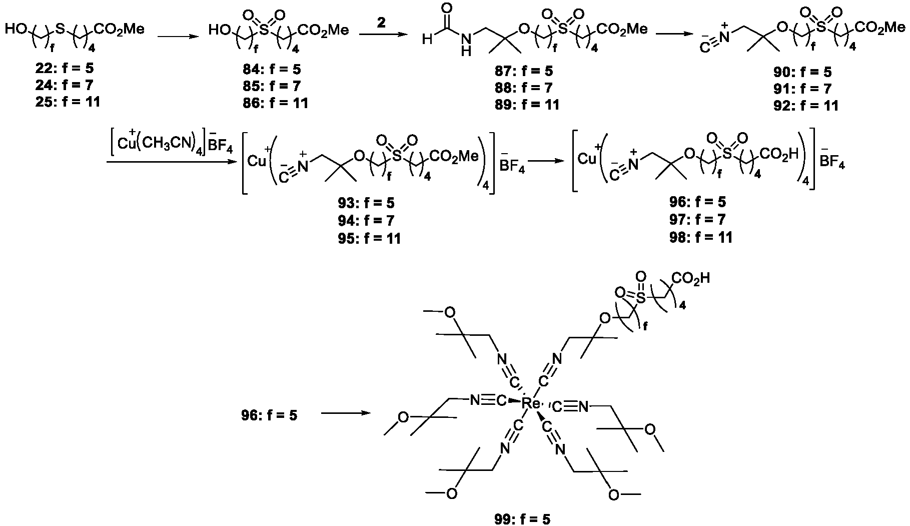
(2) The synthesis and labeling process is as follows:
   Synthesis of intermediate **84:** *m*-Chloroperoxybenzoic acid (1.0859 g) was added in batches to a dichloromethane solution of intermediate **22** (2.0846 g) described in the present application at 0 °C and stirred at room temperature for 1 h or overnight. The mixture was quenched with saturated aqueous sodium bicarbonate solution and saturated aqueous sodium thiosulfate solution, filtered, extracted with dichloromethane, and concentrated under reduced pressure to obtain intermediate 84 (1.4418 g), which was used directly in the next step without further purification.
   Synthesis of intermediate **85:** For specific operations, refer to "Synthesis of intermediate **84"** in this example. Intermediate 85 (6.8010 g) was obtained from intermediate **24** (6.3720 g) and *m*-chloroperbenzoic acid (14.3523 g). The product was directly used in the next step without further purification.
   Synthesis of intermediate **86:** For specific operations, refer to "Synthesis of intermediate **84"** in this example. Intermediate **86** (6.6374 g) was obtained from intermediate **25** (6.4185 g) and *m*-chloroperbenzoic acid (11.9103 g). The product was directly used in the next step without further purification.
   Synthesis of intermediate **87:** For specific operations, refer to "Synthesis of intermediate **29"** in Example 1. Relatively pure intermediate **87** (0.6449 g) was obtained from intermediate **2** (0.3577 g), intermediate **84** (1.4418 g), mercuric acetate (1.4261 g) and sodium borohydride (0.1447 g).
   Synthesis of intermediate **88:** For specific operations, refer to "Synthesis of intermediate **29"** in Example 1. Relatively pure intermediate **88** (1.2788 g) was obtained from intermediate **2** (1.5266 g), intermediate **85** (6.8010 g), mercuric acetate (6.0856 g) and sodium borohydride (0.6175 g). Its data characterization: HRMS: calcd 394.2263 (M+H)⁺, found 394.2410 (M+H)⁺, calcd 416.2083 (M+Na)⁺, found 416.2247 (M+Na)⁺.
   Synthesis of intermediate **89:** For specific operations, refer to the "Synthesis of intermediate **29"** in Example 1. Relatively pure intermediate **89** (1.7163 g) was obtained from intermediate **2** (1.2514 g), intermediate **86** (6.6374 g), mercuric acetate (4.9886 g) and sodium borohydride (0.5062 g).
   Synthesis of isocyanomethyl ester intermediate **90:** For the specific operation, refer to the "Synthesis of isocyanomethyl ester intermediate **39"** in Example 1. Isocyanomethyl ester intermediate **133** (0.3714 g) was obtained from intermediate **87** (0.6449 g), triethylamine (0.4946 g) and phosphorus oxychloride (0.2976 g). ¹H NMR (400 MHz, CD₃OD): δ 3.67 (s, 3H), 3.52 (s, 2H), 3.43 (t, *J =* 5.6 Hz, 2H), 3.04-3.13 (m, 4H), 2.41 (t, *J =* 7.0 Hz, 2H), 1.74-1.87 (m, 6H), 1.52-1.62 (m, 4H), 1.25 (s, 6H); ¹³C NMR (101 MHz, CD₃OD): δ 61.00, 52.03, 51.59, 32.68, 29.25, 24.92, 23.35, 21.92, 21.22, 20.93.
   Synthesis of isocyanomethyl ester intermediate **91:** For the specific operation, refer to the "Synthesis of isocyanomethyl ester intermediate **39"** in Example 1. Isocyanomethyl ester intermediate **91** (0.5546 g) was obtained from intermediate **88** (1.2788 g), triethylamine (0.9108 g) and phosphorus oxychloride (0.5480 g). ¹H NMR (400 MHz, CDCl₃): δ 3.68 (s, 3H), 3.34-3.37 (m, 4H), 2.92-2.98 (m, 4H), 2.38 (t, *J = 7.2* Hz, 2H), 1.77-1.92 (m, 6H), 1.49-1.55 (m, 2H), 1.42-1.47 (m, 2H), 1.36-1.38 (m, 4H), 1.26 (s, 6H).
   Synthesis of isocyanomethyl ester intermediate **92:** For the specific operation, refer to the "Synthesis of isocyanomethyl ester intermediate **39"** in Example 1. Isocyanomethyl ester intermediate **135** (0.5976 g) was obtained from intermediate **89** (1.7163 g), triethylamine (1.0699 g) and phosphorus oxychloride (0.6437 g). ¹H NMR (400 MHz, CDCl₃): δ 3.68 (s, 3H), 3.33-3.37 (m, 4H), 2.92-2.98 (m, 4H), 2.38 (t, *J =* 7.1 Hz, 2H), 1.77-1.92 (m, 6H), 1.49-1.56 (m, 2H), 1.44-1.45 (m, 2H), 1.28-1.37 (m, 12H), 1.27 (s, 6H).
   Synthesis of copper methyl ester intermediate **93:** For specific operations, refer to the "Synthesis of copper methyl ester intermediate **49"** in Example 1. Relatively pure copper methyl ester intermediate **93** (0.1434 g) was obtained from isocyanomethyl ester intermediate **90** (0.3714 g) and tetra(acetonitrile)copper(I) tetrafluoroborate (0.0841 g).
   Synthesis of copper salt methyl ester intermediate **94:** For specific operations, refer to the "Synthesis of copper salt methyl ester intermediate **49"** in Example 1. Relatively pure copper salt methyl ester intermediate **94** (0.2530 g) was obtained from isocyanomethyl ester intermediate **91** (0.5546 g) and tetra(acetonitrile)copper(I) tetrafluoroborate (0.1161 g). HRMS: calcd 438.1370 (M+Cu)⁺, found 438.1492 (M+Cu)⁺; calcd 813.3449 (2M+Cu)⁺, found 813.3667 (2M+Cu)⁺. When the compound is a copper salt, "M" here refers to the exact mass of the isocyanate monomer in the copper salt, and the same applies hereinafter.
   Synthesis of copper salt methyl ester intermediate **95:** For specific operations, refer to the "Synthesis of copper salt methyl ester intermediate **49"** in Example 1. Relatively pure copper salt methyl ester intermediate **95** (0.2695 g) was obtained from isocyanomethyl ester intermediate **92** (0.5576 g) and tetra(acetonitrile)copper(I) tetrafluoroborate (0.1010 g). HRMS: calcd 494.2164 (M+Cu)⁺, found 494.1996 (M+Cu)⁺; calcd 925.470 (2M+Cu)⁺, found 925.5005 (2M+Cu)⁺.
   Synthesis of copper carboxylate labeling precursor **96:** For the specific operation, refer to the "Synthesis of copper carboxylate labeling precursor **59"** in 1. Relatively pure copper carboxylate labeling precursor **96** (0.0479 g) was obtained from copper methyl ester intermediate **93** (0.1434 g) and sodium hydroxide (0.0149 g). ¹H NMR (400 MHz, CD₃OD): δ 3.74 (s, 8H), 3.43 (t, *J =* 5.9 Hz, 8H), 3.07-3.11 (m, 16H), 2.23 (t, *J =* 7.6 Hz, 8H), 1.79-1.85 (m, 16H), 1.70-1.77 (m, 8H), 1.56-1.60 (m, 16H), 1.27 (s, 24H); HRMS: calcd 396.0900 (M+Cu)⁺, found 396.0519 (M+Cu)⁺; calcd 729.2510 (2M+Cu)⁺, found 729.2190 (2M+Cu)⁺.
   Synthesis of copper carboxylate labeling precursor **97:** For the specific operation, refer to the "Synthesis of copper carboxylate labeling precursor **59"** in 1. Relatively pure copper carboxylate labeling precursor **97** (0.1134 g) was obtained from copper methyl ester intermediate **94** (0.2530 g) and sodium hydroxide (0.0245 g). HRMS: calcd 424.1213 (M+Cu)⁺, found 424.1282 (M+Cu)⁺; calcd 785.3136 (2M+Cu)⁺, found 785.3275 (2M+Cu)⁺.
   Synthesis of copper carboxylate labeling precursor **98:** For specific operations, refer to the "Synthesis of copper carboxylate labeling precursor **59"** in Example 1. Relatively pure copper carboxylate labeling precursor **98** (0.1642 g) was obtained from copper methyl ester intermediate **95** (0.2695 g) and sodium hydroxide (0.0230 g). HRMS: calcd 480.1839 (M+Cu)⁺, found 480.1938 (M+Cu)⁺; calcd 897.3888 (2M+Cu)⁺, found 897.4505 (2M+Cu)⁺.
   Synthesis of Re complex **99:** For the specific operation, refer to the "Synthesis of Re complex **69"** in Example 1, and Re complex 99 is obtained by labeling precursor **96** (1.0-3.0 mg) with copper salt carboxylic acid.HRMS: calcd 1085.5371 (M)⁺, found 1085.5135 (M)⁺_{∘}

In addition, the labeling route and method of the radioactive ^{99m}Tc complex in this embodiment are the same as the corresponding steps in the above embodiments, and will not be repeated here.

### Example 6

The organic synthesis route and method of the labeled precursors **124-126** of the typical compounds of general formula IV in this application, as well as the Re (thenium-186) complexes **127-129** of some typical compounds and their corresponding partial radioactive ^{99m}Tc (technetium-99m) complexes 130 labeling route and method (in this example, when e in general formula IV is 3, f is 5, 6, 8, and g is 4), are as follows:
(1) The synthesis and labeling route are as follows: 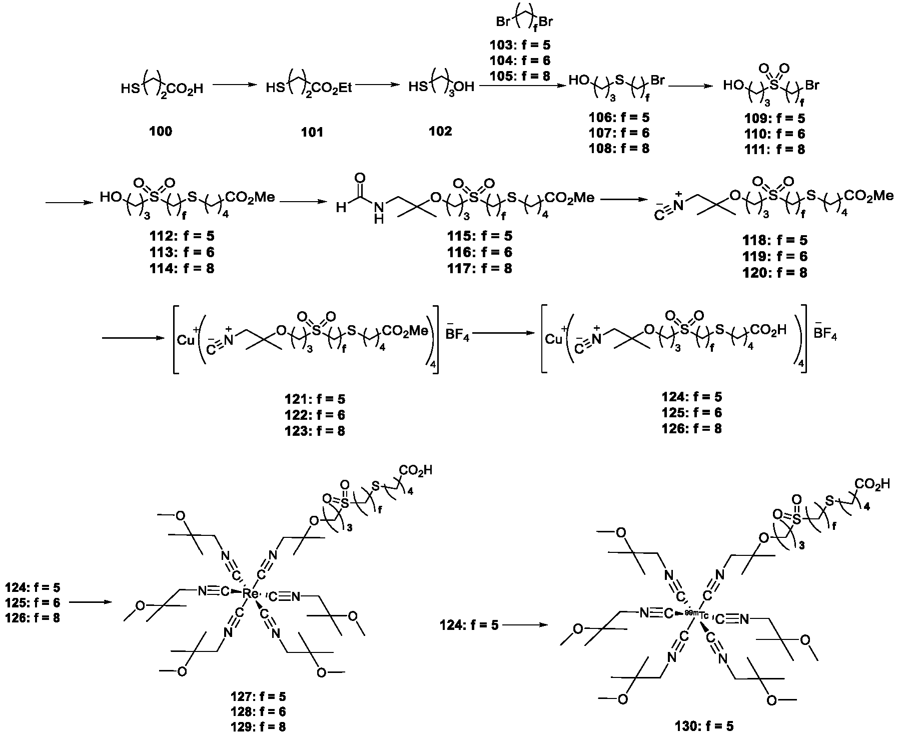
(2) The synthesis and labeling process is as follows:
   Synthesis of intermediate **101**: For the specific operation, refer to the "Synthesis of Intermediate 7" in Example 1. The intermediate **101** (213.7000 g) was obtained by reacting the raw material 100 (200.0000 g), anhydrous ethanol and *p*-toluenesulfonic acid (9.7340 g). ¹H NMR (400 MHz, CDCl₃): δ 4.17 (q, *J =* 7.1 Hz, 2H), 2.78 (q, *J =* 7.0 Hz, 2H), 2.64 (t, *J =* 6.7 Hz, 2H), 1.65 (t, *J =* 8.3 Hz, 1H), 1.27 (t, *J =* 7.2 Hz, 3H).
   Synthesis of Intermediate **102:** Lithium aluminum hydride (259.5824 g) was added in batches to a tetrahydrofuran solution of Intermediate **101** (203.4000 g) and stirred overnight at room temperature. After quenching, the mixture was extracted with ethyl acetate, and concentrated under reduced pressure to obtain Intermediate **102** (129.7912 g). The product was used directly in the next step without further purification. ¹H NMR (400 MHz, CDCl₃, CDCl₃): δ 3.77 (t, *J =* 6.1 Hz, 2H), 2.65 (q, *J =* 7.0 Hz, 2H), 1.83-1.90 (m, 2H), 1.41 (t, *J =* 8.1 Hz, 1H).
   Synthesis of intermediate **106:** Intermediate **102** (35.0000 g), raw material **103** (87.3253 g) and 1,8-diazabicycloundec-7-ene (i.e., DBU, 153.6052 g) were stirred at room temperature for 1 h. The mixture was quenched with water, extracted with dichloromethane, and concentrated under reduced pressure to obtain a crude product intermediate **106,** which was directly used in the next step without further purification.
   Synthesis of intermediate **107:** For the specific operation, refer to "Synthesis of intermediate **106".** Intermediate **102** (40.7420 g) and raw material **104** (107.8540 g) were reacted and separated by silica gel column chromatography to obtain intermediate **107** (31.9044 g). Its data characterization: ¹H NMR (400 MHz, CDCl₃): δ 3.76 (t, *J* = 5.2 Hz, 2H), 3.41 (t, *J* = 6.8 Hz, 2H), 2.64 (t, *J* = 7.0 Hz, 2H), 2.54 (t, *J* = 7.3 Hz, 2H), 1.82-1.89 (m, 4H), 1.65 (t*, J =* 7.0 Hz, 1H), 1.57-1.62 (m, 2H), 1.42-1.50 (m, 4H).
   Synthesis of intermediate **108:** For the specific operation, refer to "Synthesis of intermediate **106".** Intermediate **102** (33.0430 g) and raw material **105** (97.5299 g) were reacted and separated by silica gel column chromatography to obtain intermediate **108** (23.0627 g). Its data characterization is as follows: ¹H NMR (400 MHz, CDCl₃): δ 3.66 (t, *J =* 6.0 Hz, 2H), 3.35 (t, *J =* 6.8 Hz, 2H), 2.56 (t, *J=* 7.1 Hz, 2H), 2.46 (t, *J =* 7.6 Hz, 2H), 2.40 (t, *J =* 7.0 Hz, 1H), 1.76-1.81 (m, 4H), 1.49-1.54 (m, 2H), 1.28-1.37 (m, 8H).
   Synthesis of Intermediate **109:** For the specific operation, refer to the "Synthesis of Intermediate **84"** in Example 5. The crude Intermediate **106** (calculated according to the amount of substance of Intermediate **102)** was reacted and separated by silica gel column chromatography to obtain Intermediate **109** (22.6074 g). ¹H NMR (400 MHz, CDCl₃): δ 3.79(t, *J =* 5.8 Hz, 2H), 3.43 (t, *J =* 6.7 Hz, 2H), 3.14 (t, *J =* 7.6 Hz, 2H), 3.02 (t, *J =* 7.9 Hz, 2H), 2.80 (t, *J =* 6.2 Hz, 1H), 2.05-2.12 (m, 2H), 1.85-1.95 (m, 4H), 1.59-1.66 (m, 2H).
   Synthesis of intermediate **110:** For the specific operation, refer to the "Synthesis of intermediate **84"** in Example 5. Intermediate **110** (33.0875 g) was obtained from intermediate 107 (31.9044 g). The product was directly used in the next step without further purification.
   Synthesis of intermediate **111:** For the specific operation, refer to the "Synthesis of intermediate **84"** in Example 5. Intermediate **111** (25.1807 g) was obtained from intermediate **108** (23.0627 g). The product was directly used in the next reaction without further purification.
   Synthesis of Intermediate **112:** For the specific operation, refer to the "Synthesis of Intermediate **19"** in Example 1. Intermediate **112** (15.5740 g) was obtained from Intermediate **109** (22.6074 g). ¹H NMR (400 MHz, CDCl₃): δ 3.78 (t, *J =* 5.6 Hz, 2H), 3.67 (s, 3H), 3.13 (t, *J =* 7.4 Hz, 2H), 3.00 (t, *J =* 7.8 Hz, 2H), 2.50-2.54 (m, 5H), 2.34 (t, *J =* 7.2 Hz, 2H), 2.05-2.11 (m, 2H), 1.83-1.91 (m, 2H), 1.67-1.76 (m, 2H), 1.54-1.66 (m, 6H).
   Synthesis of intermediate **113:** For the specific operation, refer to the "Synthesis of Intermediate **19"** in Example 1. Relatively pure intermediate **113** (17.8940 g) was obtained from intermediate **110** (33.0875 g). ¹H NMR (400 MHz, CDCl₃): δ 3.81 (t, *J =* 5.6 Hz, 2H), 3.67 (s, 3H), 3.13 (t, *J =* 7.4 Hz, 2H), 2.97-3.01 (m, 2H), 2.45-2.53 (m, 4H), 2.34 (t, *J =* 7.2 Hz, 2H), 2.07-2.14 (m, 2H), 1.82-1.90 (m, 3H), 1.71-1.77 (m, 2H), 1.61-1.68 (m, 4H), 1.42-1.49 (m, 4H).
   Synthesis of intermediate **114:** For the specific operation, refer to the "Synthesis of Intermediate **19"** in Example 1. Relatively pure intermediate **114** (10.9546 g) was obtained from intermediate **111** (25.1807 g). ¹H NMR (400 MHz, CDCl₃): δ 3.73 (t, *J =* 5.9 Hz, 2H), 3.66 (s, 3H), 3.12 (t, *J =* 7.6 Hz, 2H), 2.97-3.01 (m, 2H), 2.47-2.52 (m, 4H), 2.33 (t, *J =* 7.2 Hz, 2H), 2.01-2.08 (m, 2H), 1.79-1.90 (m, 2H), 1.69-1.76 (m, 2H), 1.53-1.64 (m, 4H), 1.25-1.46 (m, 8H).
   Synthesis of Intermediate **115:** For the specific operation, refer to the "Synthesis of Intermediate **29"** in Example 1. Relatively pure Intermediate **115** (2.6092 g) was obtained from Intermediate **2** (3.0230 g) and Intermediate **112** (15.5740 g). Its data characterization is as follows: HRMS: calcd 440.2141 (M+H)⁺, found 440.2153 (M+H)⁺, calcd 462.1960 (M+Na)⁺, found 462.1957 (M+Na)⁺.
   Synthesis of Intermediate **116:** For the specific operation, refer to the "Synthesis of Intermediate **29"** in Example 1. Relatively pure Intermediate **116** (2.3764 g) was obtained from Intermediate **2** (3.3357 g) and Intermediate **113** (17.8940 g). HRMS: calcd 454.2297 (M+H)⁺, found 454.2280 (M+H)⁺, calcd 476.2116 (M+Na)⁺, found 476.2101 (M+Na)⁺.
   Synthesis of Intermediate **117:** For the specific operation, refer to the "Synthesis of Intermediate **29"** in Example 1. Relatively pure Intermediate **117** (1.0537 g) was obtained from Intermediate **2** (1.8920 g) and Intermediate **114** (10.9546 g). Its data characterization is as follows: HRMS: calcd 482.2610 (M+H)⁺, found 482.2572 (M+H)⁺, calcd 504.2429 (M+Na)⁺, found 504.2376 (M+Na)⁺.
   Synthesis of isocyanomethyl ester intermediate **118:** For specific operations, refer to the "Synthesis of isocyanomethyl ester intermediate **39"** in Example 1. Isocyanomethyl ester intermediate **118** (869.4 mg) was obtained from intermediate **115** (2.6092 g). ¹H NMR (400 MHz, CDCl₃): δ 3.67 (s, 3H), 3.53 (t, *J =* 5.7 Hz, 2H), 3.38 (s, 2H), 3.11 (t, *J =* 7.4 Hz, 2H), 2.99 (t, *J =* 7.9 Hz, 2H), 2.49-2.54 (m, 4H), 2.34 (t, *J =* 7.2 Hz, 2H), 2.06-2.13 (m, 2H), 1.83-1.91 (m, 2H), 1.67-1.77 (m, 2H), 1.53-1.64(m, 6H), 1.28(s, 6H).
   Synthesis of isocyanomethyl ester intermediate **119:** For specific operations, refer to the "Synthesis of isocyanomethyl ester intermediate **39"** in Example 1. Isocyanomethyl ester intermediate **119** (956.2 mg) was obtained from intermediate **116** (2.3764 g). ¹H NMR (400 MHz, CDCl₃): δ 3.60 (s, 3H), 3.46 (t, *J =* 5.7 Hz, 2H), 3.31 (s, 2H), 3.03 (t, *J =* 7.4 Hz, 2H), 2.89-2.93 (m, 2H), 2.42-2.46 (m, 4H), 2.27 (t, *J =* 7.2 Hz, 2H), 2.00-2.06 (m, 2H), 1.75-1.83 (m, 2H), 1.63-1.70 (m, 2H), 1.47-1.58 (m, 4H), 1.36-1.39 (m, 4H), 1.28(s, 6H).
   Synthesis of isocyanomethyl ester intermediate **120:** For specific operations, refer to the "Synthesis of isocyanomethyl ester intermediate **39"** in Example 1. Isocyanomethyl ester intermediate **120** (675.7 mg) was obtained from intermediate **117** (1.0537 g). ¹H NMR (400 MHz, CDCl₃): δ 3.60 (s, 3H), 3.46 (t, *J =* 5.6 Hz, 2H), 3.31 (s, 2H), 3.03 (t, *J=* 7.4 Hz, 2H), 2.90 (t, *J =* 7.8 Hz, 2H), 2.41-2.46 (m, 4H), 2.27 (t, *J =* 7.3 Hz, 2H), 1.99-2.06 (m, 2H), 1.73-1.81 (m, 2H), 1.63-1.70 (m, 2H), 1.46-1.58 (m, 4H), 1.26-1.39 (m, 8H), 1.21 (s, 6H).
   Synthesis of copper salt methyl ester intermediate **121:** For specific operations, refer to the "Synthesis of copper salt methyl ester intermediate **49"** in Example 1. Relatively pure copper salt methyl ester intermediate **121** (742.5 mg) was obtained from isocyanomethyl ester intermediate **118** (869.4 mg). Its data characterization is as follows: HRMS: calcd 484.1247 (M+Cu)⁺, found 484.0943 (M+Cu)⁺.When the compound is a copper salt, "M" here refers to the exact mass of the isocyanate monomer in the copper salt, the same below.
   Synthesis of copper salt methyl ester intermediate **122:** For specific operations, refer to the "Synthesis of copper salt methyl ester intermediate **49"** in Example 1. A relatively pure copper salt methyl ester intermediate **165** (344.0 mg) was obtained from isocyanomethyl ester intermediate **119** (956.2 mg). HRMS: calcd 498.1404 (M+Cu)⁺, found 498.1386 (M+Cu)⁺.
   Synthesis of copper salt methyl ester intermediate **123:** For specific operations, refer to the "Synthesis of copper salt methyl ester intermediate **49"** in Example 1. Relatively pure copper salt methyl ester intermediate 166 (499.1 mg) was obtained from isocyanomethyl ester intermediate **120** (675.7 mg). Its data characterization is as follows: HRMS: calcd 526.1717 (M+Cu)⁺, found 526.1777 (M+Cu)⁺.
   Synthesis of copper carboxylate labeling precursor **124:** For specific operations, refer to the "Synthesis of copper carboxylate labeling precursor **59"** in Example 1. Relatively pure copper carboxylate labeling precursor **124** (205.0 mg) was obtained from copper methyl ester intermediate **121** (742.5 mg). HRMS: calcd 470.1091 (M+Cu)⁺, found 470.1076 (M+Cu)⁺.
   Synthesis of copper carboxylate labeling precursor **125:** For specific operations, refer to the "Synthesis of copper carboxylate labeling precursor **59"** in Example 1. Relatively pure copper carboxylate labeling precursor **125** (176.0 mg) was obtained from copper methyl ester intermediate **122** (344.0 mg). HRMS: calcd 484.1247 (M+Cu)⁺, found 484.1274 (M+Cu)⁺.
   Synthesis of copper carboxylate labeling precursor **126:** For specific operations, refer to the "Synthesis of copper carboxylate labeling precursor **59"** in Example 1. Relatively pure copper carboxylate labeling precursor **126** (238.1 mg) was obtained from copper methyl ester intermediate **123** (499.1 mg). Its data characterization is as follows: HRMS: calcd 512.1560 (M+Cu)⁺, found 512.1562 (M+Cu)⁺.
   Synthesis of Re complex **127:** For specific operations, refer to "Labeling of Re complex **69"** in Example 1. Precursor **124** (1.2 ~ 3.5 mg) was labeled with copper salt carboxylic acid to obtain Re complex **127.** HRMS: calcd 1159.5528 (M)⁺, found 1159.5609 (M)⁺
   Synthesis of Re complex **128:** For specific operations, refer to "Labeling of Re complex **69"** in Example 1. Precursor **125** (1.2 ~ 3.7 mg) was labeled with copper salt carboxylic acid to obtain Re complex **128.** HRMS: calcd 1173.5684 (M)⁺, found 1173.5747 (M)⁺
   Synthesis of Re complex **129:** For specific operations, refer to "Labeling of Re complex **69"** in Example 1. Precursor **126** (1.3 ~ 3.9 mg) was labeled with copper salt carboxylic acid to obtain Re complex **129,** which was characterized by the following data: HRMS: calcd 1201.5997 (M)⁺, found 1201.6062 (M)⁺
   Synthesis of ^{99m}Tc complex **130:** For specific operations, refer to "Labeling of ^{99m}Tc complex **78"** in Example 1. Precursor **124** (1.2 ~ 3.5 mg) was labeled with copper salt carboxylic acid to obtain ^{99m}Tc complex **130.** The HPLC spectrum of co-injection of ^{99m}Tc complex **130** and the corresponding Re complex **127** (methanol/water containing 1‰ trifluoroacetic acid = 70/30, total flow rate 1.0 mL/min, C-18 reverse phase semi-preparative column) was shown in Fig. 9. The retention time of ^{99m}Tc radioactive complex **130** (75.075 min) was basically consistent with the retention time of the corresponding Re complex **127** (76.425 min).

### Example 7

The corresponding radioactive ^{99m}Tc complex **130** as the typical compound of the general formula IV in this application was selected for *in vivo* biodistribution study in female Kunming mice, as shown below:
(1) Experimental method
Female Kunming mice (20-22 g, n = 3) were fasted for 12 h before the experiment, and the purified radioactive ^{99m}Tc complex **130** was dissolved in a normal saline (containing 10% ethanol) solution (about 100 µCi/mL). Female Kunming mice were injected with 100 µL of the above solution through the tail vein, and the mice were killed by cervical dislocation at 5, 15, 30, 60, and 120 min post-injection, respectively. Blood, brain, heart, liver, spleen, lung, kidney, muscle, bone, large intestine, small intestine, stomach and tail were collected, weighed and counted, and the count distribution of each tissue and organ was calculated (unit: %ID/g, the unit of large intestine, small intestine and stomach was %ID), and the data were the mean ± standard deviation of three mice in each time phase. At the same time, 100 µL of the above solution was diluted to 10 mL, which was used as the %ID without deducting the tail count.
(2) The biodistribution results of radioactive ^{99m}Tc complex **130** in female Kunming mice were as follows:

**Table 5 Biodistribution data of radioactive ^{99m}Tc complex 130 in female Kunming mice (n=3, ID%/g)**

| Organ/Tissue | Time (min) | | | | |
|---|---|---|---|---|---|
| | 5 | 15 | 30 | 60 | 120 |
| Blood | 0.54±0.07 | 0.24±0.01 | 0.12±0.04 | 0.08±0.01 | 0.04±0.01 |
| Brain | 0.04±0.00 | 0.03±0.01 | 0.02±0.01 | 0.02±0.00 | 0.02±0.01 |
| Heart | 2.14±0.25 | 1.92±0.18 | 1.54±0.38 | 1.37±0.26 | 1.27±0.03 |
| Liver | 53.13±4.86 | 42.04±2.91 | 18.08±0.30 | 15.12±1.7 | 3.74±0.98 |
| Spleen | 0.67±0.04 | 0.50±0.03 | 0.31±0.05 | 0.14±0.03 | 0.13±0.02 |
| Lungs | 0.84±0.09 | 0.76±0.03 | 0.28±0.05 | 0.19±0.01 | 0.19±0.05 |
| Kidney | 9.64±0.08 | 7.89±0.19 | 2.51±0.58 | 1.20±0.10 | 0.30±0.04 |
| Muscle | 0.75±0.06 | 0.67±0.07 | 0.59±0.16 | 0.43±0.09 | 0.38±0.10 |
| Bone | 0.54±0.10 | 0.76±0.13 | 0.33±0.12 | 0.15±0.03 | 0.09±0.01 |
| Large Intestine | 0.53±0.10 | 0.82±0.34 | 1.56±0.16 | 16.13±5.3 | 34.11±4.52 |
| Small Intestine | 4.32±0.25 | 20.74±0.83 | 29.63±3.26 | 26.18±3.9 | 7.50±2.45 |
| Stomach | 0.17±0.02 | 0.26±0.01 | 0.12±0.04 | 0.29±0.05 | 0.17±0.12 |
| Heart-to-Blood | 3.96 | 8.00 | 12.83 | 17.13 | 31.75 |
| Heart-to-Liver | 0.04 | 0.05 | 0.09 | 0.09 | 0.34 |
| Heart-to-Lungs | 2.55 | 2.53 | 5.50 | 7.21 | 6.68 |
| Heart-to-Muscle | 2.85 | 2.87 | 2.61 | 3.19 | 3.34 |

The absolute myocardial uptake of radioactive ^{99m}Tc complex **130** was relatively low after tail vein injection.

### Example 8

In addition, the structures of the typical compounds of the general formula IV in the present application - its isocyanomethyl ester intermediate **131,** its copper salt carboxylic acid labeling precursor **132,** and its corresponding Re complex **133** are shown below (in this embodiment, when e in the general formula IV is 6, f is 5, and g is 4), and its organic synthesis route and method, labeling route and method are completely consistent with those in the above-mentioned Example 7. The final steps are also respectively referred to the "Synthesis of isocyanomethyl ester intermediate **39",** "Synthesis of copper salt carboxylic acid labeling precursor **59",** and "Labeling of Re complex **69"** in Example 1. The specific process will not be repeated here.

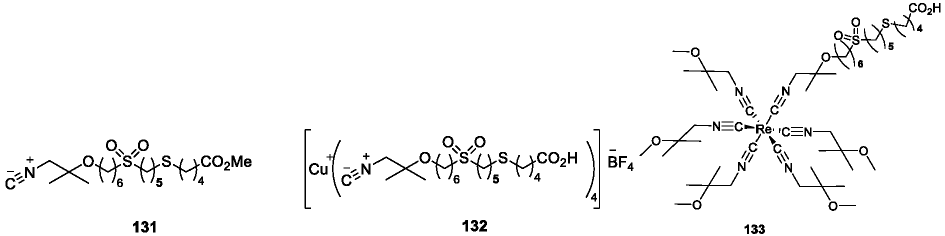

Isocyanomethyl ester intermediate **131.** ¹H NMR (400 MHz, CDCl₃): δ 3.67 (s, 3H), 3.34-3.38 (m, 4H), 2.93-2.97 (m, 4H), 2.49-2.54 (m, 4H), 2.34 (t, *J =* 7.8 Hz, 2H), 1.82-1.90 (m, 4H), 1.67-1.77 (m, 2H), 1.42-1.63 (m, 12H), 1.27 (s, 6H).

Isocyanomethyl ester intermediate **132.** HRMS: calcd 512.1560 (M+Cu)⁺, found 512.1545 (M+Cu)⁺. When the compound is a copper salt, "M" herein refers to the exact mass of isocyanate monomers in the copper salt.
Re complex **133.** HRMS: calcd 1201.6030 (M)⁺, found 1201.5981 (M)⁺

### Example 9

In addition, the synthetic routes and methods of intermediates **97-98** of the labeled precursors of typical compounds of general formula IV in this application (in this embodiment, when e in general formula IV is 2, f is 2, and g is 2 and 4 respectively) are as follows:
(1) The synthetic and labeling routes are as follows: 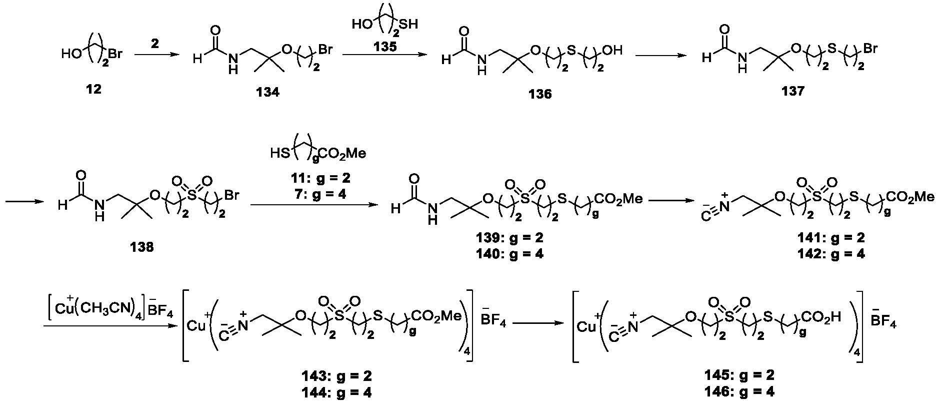 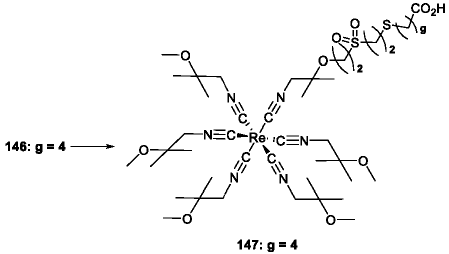
(2) The synthesis method is as follows:
   Synthesis of intermediate **134:** For the specific operation, refer to the "Synthesis of intermediate **29"** in Example 1 above. Relatively pure intermediate **134** (51.7092 g) was obtained from intermediate **2** (39.6520 g) and raw material **12** (74.9760 g), which was directly used in the next step reaction without further purification.
   Synthesis of Intermediate **136:** For the specific operation, refer to the "Synthesis of Intermediate **106"** in Example 6 above. Relatively pure intermediate **134** (47.5935 g), 2-mercaptoethanol **(135,** 18.2522 g) and DBU (39.2512 g) were reacted and separated by silica gel column chromatography to obtain relatively pure intermediate **136** (11.0177 g). ¹H NMR (400 MHz, CDCl₃): δ 8.25 (s, 1H), 6.52 (brs, 1H), 3.75-3.79 (m, 3H), 3.54 (t, *J =* 5.8 Hz, 2H), 3.35 (d, *J* = 6.0 Hz, 2H), 2.79 (t, *J =* 5.8 Hz, 2H), 2.74 (t, *J =* 5.8 Hz, 1H), 1.21 (s, 6H).
   Synthesis of Intermediate 137: Phosphorus tribromide (14.9029 g) was added to a relatively pure solution of Intermediate **136** (11.0177 g) in dichloromethane and stirred at room temperature overnight. The mixture was then quenched with saturated aqueous sodium bicarbonate solution, extracted with dichloromethane, dried, and concentrated *in vacuo* to obtain a concentrate containing Intermediate **137** (9.3624 g), which was used directly in the next step without further purification.
   Synthesis of intermediate **138:** For the specific operation, refer to the "Synthesis of intermediate **84"** in Example 5 above. A residue containing intermediate **138** (10.4163 g) was obtained from the residue containing intermediate **137** (9.3624 g). The product was directly used in the next step without further purification.
   Synthesis of Intermediate **139:** For the specific operation, refer to the "Synthesis of Intermediate **19"** in Example 1 above. Intermediate **138** (3.5466 g) was reacted with raw material **11** (1.6174 g) to obtain Intermediate **139** (2.8267 g). ¹H NMR (400 MHz, CDCl₃): δ 8.22 (s, 1H), 6.93 (brs, 1H), 3.82 (t, *J =* 5.3 Hz, 2H), 3.24-3.50 (m, 6H), 2.93-2.99 (m, 2H), 2.84 (t, *J =* 7.1 Hz, 2H), 2.65 (t, *J =* 7.1 Hz, 2H), 1.22 (s, 6H); HRMS: calcd 356.1202 (M+H)⁺, found 356.1206 (M+H)⁺, calcd 378.1021 (M+Na)⁺, found 378.1010 (M+Na)⁺.
   Synthesis of Intermediate **140:** For the specific operation, refer to the "Synthesis of Intermediate **19"** in Example 1 above. Intermediate **138** (3.2899 g) and Intermediate 7 (1.8505 g) were reacted to obtain Intermediate **140** (2.6050 g). ¹H NMR (400 MHz, CDCl₃): δ 8.23 (s, 1H), 6.82 (brs, 1H), 3.82 (t, J = 5.2 Hz, 2H), 3.32-3.43 (m, 4H), 3.30 (t, *J=* 5.4 Hz, 2H), 2.87-2.98 (m, 2H), 2.59 (t, *J =* 7.1 Hz, 2H), 2.35 (t, *J =* 7.1 Hz, 2H), 1.69-1.77 (m, 2H), 1.59-1.67 (m, 2H), 1.22 (s, 6H); HRMS: calcd 384.1515 (M+H)⁺, found 384.1450 (M+H)⁺, calcd 406.1334 (M+Na)⁺, found 406.1317 (M+Na)⁺.
   Synthesis of isocyano monomer methyl ester intermediate **141:** For specific operations, refer to the "Synthesis of Intermediate **39"** in Example 1 above. Intermediate **139** (2.0180 g) was obtained from Intermediate **139** (2.8267 g). ¹H NMR (400 MHz, CDCl₃): δ 3.78 (t, *J =* 5.3 Hz, 2H), 3.64 (s, 3H), 3.29-3.39 (m, 4H), 3.19 (t, *J* = 5.3 Hz, 2H), 2.87-2.94 (m, 2H), 2.78 (t, *J =* 7.2 Hz, 2H), 2.57 (t, *J =* 7.2 Hz, 2H), 1.26 (s, 6H); ¹³C NMR (101 MHz, CDCl₃): δ 171.04, 157.39, 73.41, 59.37, 55.66, 54.26, 53.06, 50.90, 49.78, 49.71, 49.65, 33.32, 25.98, 22.97, 21.69, 20.04, 13.19.
   Synthesis of isocyano monomer methyl ester intermediate **142:** For specific operations, refer to the "Synthesis of Intermediate **39"** in Example 1 above. Intermediate **140** (1.3060 g) was obtained from Intermediate **140** (2.6050 g). ¹H NMR (400 MHz, CDCl₃): δ 3.85 (t, *J =* 5.3 Hz, 2H), 3.67 (s, 3H), 3.34-3.45 (m, 4H), 3.26 (t, *J* = 5.3 Hz, 2H), 2.89-2.98 (m, 2H), 2.59 (t, *J =* 7.1 Hz, 2H), 2.34 (t, *J =* 7.2 Hz, 2H), 1.69-1.79 (m, 2H), 1.59-1.68 (m, 2H), 1.33 (s, 6H); ¹³C NMR (101 MHz, CDCl₃): δ 173.65, 158.36, 74.34, 56.62, 55.37, 53.94, 51.54, 50.68, 50.61, 50.55, 33.43, 31.76, 28.61, 23.90, 23.81, 22.70.
   Synthesis of copper salt methyl ester intermediate **143:** For specific operations, refer to the "Synthesis of copper salt methyl ester intermediate **49"**in Example 1 above, and relatively pure copper salt methyl ester intermediate **143** (1.5498 g) was obtained from isocyanate monomer intermediate **141** (2.0180 g).
   Synthesis of copper salt methyl ester intermediate **144:** For specific operations, refer to the "Synthesis of copper salt methyl ester intermediate 49 "in Example 1 above, and relatively pure copper salt methyl ester intermediate **144** (1.0444 g) was obtained from isocyano monomer intermediate 142 (1.3060 g).
   Synthesis of copper carboxylate labeling precursor **145:** For the specific operation, refer to the "Synthesis of copper carboxylate labeling precursor **59"** in Example 1 above. A relatively pure copper carboxylate labeling precursor **145** (0.3596 g) was obtained from the copper methyl ester intermediate **143** (1.5498 g). ¹H NMR (400 MHz, CD₃OD) δ 3.74-3.93 (m, 16H), 3.44-3.53 (m, 8H), 3.35-3.40 (m, 8H), 2.95- 3.05 (m, 8H), 2.86 (t, *J =* 7.4 Hz, 8H), 2.47 (t, *J =* 7.4 Hz, 8H), 1.32 (s, 24H).
   Synthesis of copper salt carboxylic acid labeling precursor **146:** For specific operations, refer to the "Synthesis of copper salt carboxylic acid labeling precursor **59"** in Example 1 above. Relatively pure copper salt carboxylic acid labeling precursor **146** (0.3856 g) was obtained from copper salt methyl ester intermediate **144** (1.0444 g). HRMS: calcd 414.0465 (M+Cu)⁺, found 414.0432 (M+Cu)⁺.When the compound is a copper salt, "M" herein refers to the exact mass of isocyanate monomers in the copper salt.
   Synthesis of Re complex **147:** For the specific operation, refer to the "Synthesis of Re complex **69"** in Example 1 above. Re complex **147** was obtained by labeling precursor **146** (1.0 ~ 3.1 mg) with copper salt carboxylic acid. HRMS: calcd 1103.4935 (M)⁺, found 1103.4958 (M)⁺

In addition, the labeling route and method of the radioactive ^{99m}Tc complex (in this embodiment, when e in general formula IV is 2, f is 2, g is 2 and 4 respectively, and M is ^{99m}Tc) are the same as the corresponding steps in the above embodiments and will not be repeated here.

### Example 10

The organic synthesis route and method of the labeled precursor **108** of the typical compound of general formula V in this application, as well as the labeling route and method of the Re complex **109** of the typical compound and its corresponding radioactive ^{99m}Tc complex **110** (in this example, d is 3, e is 5, f is 3, and g is 4 in general formula V) are as follows:
(1) The synthesis and labeling route are as follows: 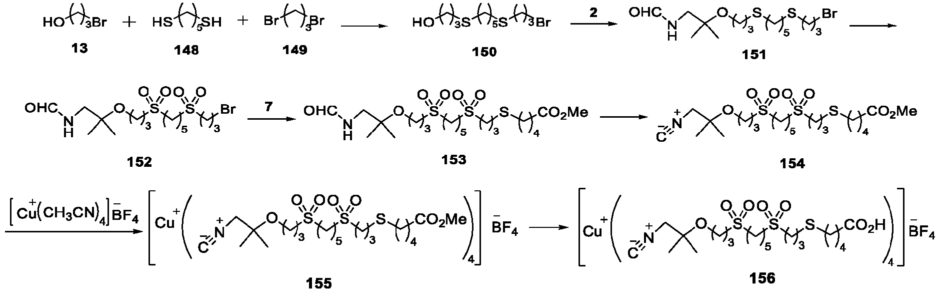 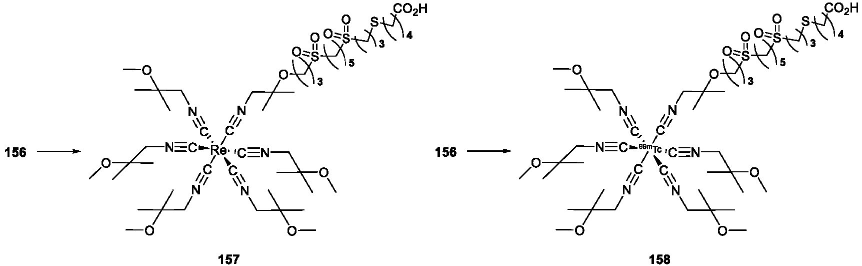
(2) The synthesis and labeling methods are as follows:
   Synthesis of intermediate **150:** For the specific operation, refer to the "Synthesis of intermediate **106"** in Example 6 above. Raw material **13** (96.8007 g), raw material **148** (94.9063 g), raw material **149** (140.6079 g) and DBU (257.4380 g) were reacted and separated by silica gel column chromatography to obtain relatively pure intermediate **150** (69.7249 g). The product was directly used in the next reaction without further purification.
   Synthesis of intermediate **151:** For the specific operation, refer to the" Synthesis of intermediate **29"in** Example 1 above. Intermediate **2** (14.6129 g) and relatively pure intermediate **150** (69.7249 g) were reacted and separated by silica gel column chromatography to obtain relatively pure intermediate **151** (17.2080 g). The product was directly used in the next reaction without further purification.
   Synthesis of Intermediate **152:** Relatively pure Intermediate **151** (17.2080 g) was dissolved in a mixed solvent of 1,4-dioxane and water, and then oxone (potassium peroxymonosulfonate, 118.0222 g) was added thereto in batches and stirred at room temperature overnight. Then, the mixture was quenched with water, extracted with dichloromethane, dried, concentrated in vacuo, and separated by silica gel column chromatography to obtain Intermediate **152** (2.4834 g). ¹H NMR (400 MHz, CDCl₃): δ 8.24 (brs, 1H), 6.25 (brs, 1H), 3.56 (t, *J =* 6.2 Hz, 2H), 3.49 (t, *J =* 5.7 Hz, 2H), 3.32 (d, *J =* 6.0 Hz, 2H), 3.00-3.18 (m, 8H), 2.38-2.44 (m, 2H), 2.05-2.11 (m, 2H), 1.89-1.97 (m, 4H), 1.64-1.72 (m, 2H), 1.17 (s, 6H).
   Synthesis of Intermediate **153:** For the specific operation, refer to the "Synthesis of Intermediate **19"** in Example 1 above. Intermediate **152** (2.4834 g) was reacted with Intermediate 7 (0.9231 g) to obtain Intermediate **153** (1.5640 g). ¹H NMR (400 MHz, CDCl₃): δ 8.23 (brs, 1H), 6.45 (brs, 1H), 3.67 (s, 3H), 3.50 (t, *J =* 5.6 Hz, 2H), 2.99-3.18 (m, 8H), 2.67 (t, *J =* 6.7 Hz, 2H), 2.53 (t, *J =* 7.1 Hz, 2H), 2.34 (t, *J =* 7.3 Hz, 2H), 2.05-2.16 (m, 4H), 1.89-1.95 (m, 4H), 1.59-1.77 (m, 8H), 1.18 (s, 6H).
   Synthesis of isocyanomethyl ester intermediate **154:** For the specific operation, refer to the "Synthesis of isocyanomethyl ester intermediate **39"** in Example 1 above. Isocyanomethyl ester intermediate **154** (1.3280 g) was obtained from intermediate **153** (1.7249 g). ¹H NMR (400 MHz, CDCl₃): δ 3.67 (s, 3H), 3.53 (t, *J =* 5.6 Hz, 2H), 3.38 (s, 2H), 3.09-3.14 (m, 4H), 2.98-3.03 (m, 4H), 2.67 (t, *J=* 6.9 Hz, 2H), 2.53 (t, *J =* 7.2 Hz, 2H), 2.34 (t, *J =* 7.1 Hz, 2H), 2.07-2.16 (m, 4H), 1.88-1.96 (m, 4H), 1.60-1.75 (m, 6H), 1.28 (s, 6H).
   Synthesis of copper salt methyl ester intermediate **155:** For specific operations, refer to the" Synthesis of copper salt methyl ester intermediate **49"**in Example 1 above, and relatively pure copper salt methyl ester intermediate **155** (1.0637 g) was obtained from isocyanomethyl ester intermediate **154** (1.3280 g).
   Synthesis of copper carboxylate labeling precursor 156: For the specific operation, refer to the "Synthesis of copper carboxylate labeling precursor **59"** in Example 1 above. A relatively pure copper carboxylate labeling precursor **156** (0.4610 g) was obtained from the copper methyl ester intermediate **155** (1.0637 g). ¹H NMR (400 MHz, CD₃OD): δ 3.79 (s, 8H), 3.56 (t, *J* = 5.2 Hz, 8H), 3.13-3.31 (m, 32H), 2.69 (t, *J* = 6.8 Hz, 8H), 2.56 (t, *J* = 7.1 Hz, 8H), 2.17 (t, *J* = 6.7 Hz, 8H), 1.94-2.11 (m, 16H), 1.80-1.93 (m, 16H), 1.55-1.73 (m, 24H), 1.29 (s, 24H); ¹³C NMR (101 MHz, CD₃OD): δ 181.16, 154.10, 73.12, 59.65, 51.76, 51.44, 50.87, 49.56, 37.31, 31.16, 29.94, 29.31, 26.83, 25.53, 22.97, 22.04, 21.63, 21.12, 21.06; ¹⁹F NMR (376 MHz, CD₃OD): δ -155.4; HRMS: calcd 576.1179 (M+Cu)⁺, found 576.1184 (M+Cu)⁺.
   Labeling of Re complex 157: For specific operations, refer to "labeling of Re complex 69" in Example 1 above, and Re complex 157 is obtained by labeling precursor 156 with copper salt carboxylic acid; HRMS: calcd 1265.5649 (M)⁺, found 1265.5552 (M)⁺
   Labeling of ^{99m}Tc complex **158:** For specific operations, refer to the "Labeling of ^{99m}Tc complex **78"**in Example 1 above, and the ^{99m}Tc radioactive complex **158** is obtained by labeling the precursor **156** with copper salt carboxylic acid. The HPLC spectrum of the co-injection of the ^{99m}Tc radioactive complex **158** and the corresponding Re complex **157** (methanol/water containing 1‰ trifluoroacetic acid = 67.5/32.5, total flow rate 1.0 mL/min, C-18 reverse phase semi-preparative column) is shown in Fig. 10, and the retention time of the ^{99m}Tc radioactive complex **158** (62.125 min) is basically consistent with the retention time of the corresponding Re complex **157** (63.192 min).

### Example 11

The corresponding radioactive ^{99m}Tc complex **158** as the typical compounds of the general formula V in this application was selected to conduct an *in vivo* biodistribution study in female Kunming mice. The specific experimental method was shown in the experimental method of "*In vivo* biodistribution study of radioactive ^{99m}Tc complex **130** in female Kunming mice" in Example 7 above. The results were as follows:

**Table 6 Biodistribution data of radioactive ^{99m}Tc complex 158 in female Kunming mice (n=3, ID%/g)**

| Organ/Tissue | Time (min) | | | | |
|---|---|---|---|---|---|
| | 5 | 15 | 30 | 60 | 120 |
| Blood | 13.21±2.84 | 5.47±0.09 | 1.20±0.1 | 0.42±0.03 | 0.26±0.01 |
| Brain | 0.39±0.14 | 0.14±0.05 | 0.1±0.04 | 0.06±0.01 | 0.03±0.02 |
| Heart | 4.19±0.10 | 1.63±0.02 | 0.91±0.11 | 0.67±0.09 | 0.52±0.01 |
| Liver | 37.80±6.80 | 33.35±0.37 | 22.5±3.2 | 22.8±1.20 | 13.81±0.59 |
| Spleen | 1.55±0.01 | 0.86±0.28 | 0.55±0.1 | 0.24±0.06 | 0.15±0.07 |
| Lungs | 5.91±0.89 | 2.69±0.70 | 0.9±0.04 | 0.50±0.02 | 0.32±0.06 |
| Kidney | 7.80±1.46 | 4.59±0.68 | 2.22±0.2 | 1.20±0.10 | 0.80±0.07 |
| Muscle | 1.82±0.16 | 1.10±0.22 | 0.46±0.1 | 0.31±0.06 | 0.21±0.03 |
| Bone | 2.31±0.36 | 1.04±0.38 | 0.37±0.1 | 0.24±0.04 | 0.15±0.05 |
| Large Intestine | 1.04±0.37 | 0.67±0.15 | 1.25±0.1 | 1.31±0.27 | 1.02±0.22 |
| Small Intestine | 5.39±0.84 | 16.67±2.60 | 20.9±4.6 | 34.24±3.12 | 19.83±1.14 |
| Stomach | 0.54±0.19 | 0.46±0.13 | 0.25±0.1 | 0.38±0.05 | 0.47±0.10 |
| Heart-to-Blood | 0.32 | 0.30 | 0.76 | 1.60 | 2.00 |
| Heart-to-Liver | 0.11 | 0.05 | 0.04 | 0.03 | 0.04 |
| Heart-to-Lungs | 0.71 | 0.61 | 1.00 | 1.34 | 1.63 |

The absolute myocardial uptake of the radioactive ^{99m}Tc complex **158** was low, the myocardial clearance was fast, and the heart-to-blood and heart-to-lungs ratios of the radiotracer were at a moderate level.

### Example 12

In addition, the synthetic route and method of the intermediate **163** of the labeled precursor of the typical compound of general formula **VI** in this application (in this embodiment, when e is 2, f is 2, and g is 4 respectively in general formula VI) are as follows:
(1) The synthetic route is as follows: 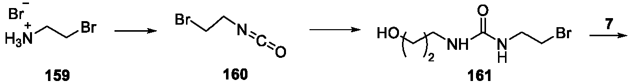 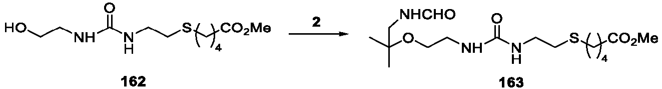
(2) The synthesis method is as follows:
   Synthesis of intermediate **160:** After adding pyridine (3.1456 g) to a dichloromethane solution of raw material **159** (2.5090 g), the mixture was cooled to -30 °C. Then, triphosgene (1.2998 g) was added to the above mixture, and then stirred in an ice bath for 4 h. The reaction solution was quenched with hydrochloric acid, extracted with dichloromethane, dried, and concentrated *in vacuo* to obtain a concentrate containing intermediate **160** (1.4693 g). The product was used directly in the next step without further purification.
   Synthesis of Intermediate **161:** 2-aminoethanol (0.4603 g) and DIPEA (diisopropylethylamine, 1.9586 g) were added to a dichloromethane solution containing a concentrate of Intermediate **160** (1.4693 g) in an ice bath. The resulting mixture was stirred for 0.5 h in an ice bath, and then stirred at room temperature overnight. The mixture was concentrated *in vacuo* and separated by silica gel column chromatography to obtain Intermediate **161** (0.9288 g). ¹H NMR (400 MHz, CD₃OD): δ 4.84 (t, *J =* 8.4 Hz, 2H), 3.94 (t, *J =* 8.6 Hz, 2H), 3.69 (t, *J =* 5.0 Hz, 2H), 3.41 (t, *J =* 5.0 Hz, 2H).
   Synthesis of Intermediate **162:** For the specific operation, refer to the "Synthesis of Intermediate **19"** in Example 1 above. Intermediate **161** (0.9288 g) was reacted with Intermediate 7 (0.7827 g) to obtain Intermediate **162** (122.3 mg). ¹H NMR (400 MHz, CD₃OD): δ 3.65 (s, 3H), 3.56 (t, *J =* 5.6 Hz, 2H), 3.28 (t, *J =* 7.2 Hz, 2H), 3.22 (t, *J =* 5.6 Hz, 2H), 2.53-2.61 (m, 4H), 2.35 (t, *J =* 7.2 Hz, 2H), 1.67-1.74 (m, 2H), 1.57-1.63 (m, 2H).
   Synthesis of Intermediate **163:** For the specific operation, refer to the "Synthesis of Intermediate **29"** in Example 1 above. After the reaction of Intermediate **2** (29.0 mg) and Intermediate 162 (122.3 mg), a concentrate containing Intermediate **163** (147.5 mg) was obtained. HRMS: calcd 378.2063 (M+H)⁺, found 378.1202 (M+H)⁺, calcd 400.1882 (M+Na)⁺, found 400.1918 (M+Na)⁺.

In addition, the subsequent intermediate **163** is converted into the corresponding copper salt carboxylic acid labeling precursor via the corresponding isocyanate monomer methyl ester intermediate and the subsequent copper salt methyl ester intermediate; and the corresponding rhenium Re complex (in this embodiment, when e is 2, f is 2, g is 4 respectively, and M is Re) and radioactive ^{99m}Tc complex (in this embodiment, when e is 2, f is 2, g is 4 respectively, and M is ^{99m}Tc in the general formula VI) labeling route and method are the same as the corresponding steps in the above embodiments, and will not be repeated here.

### Example 12

The synthetic route and method of the labeled precursor **180** of the typical compound of general formula **VII** in this application (in this example, when f is 12 and g is 1 in general formula **VII),** in addition, after the synthetic route of the labeled precursor **180,** there is also a method for increasing the g value when the value of f is relatively small, as shown below:
(1) The synthetic route is as follows: 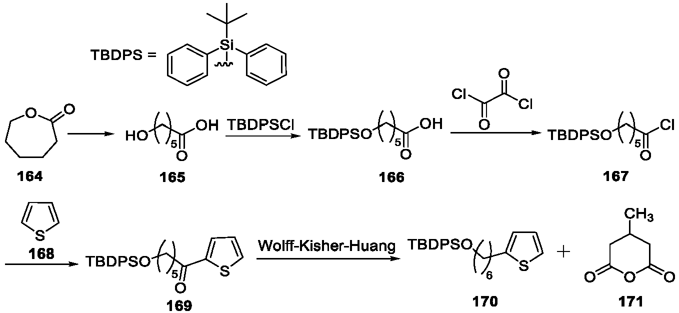 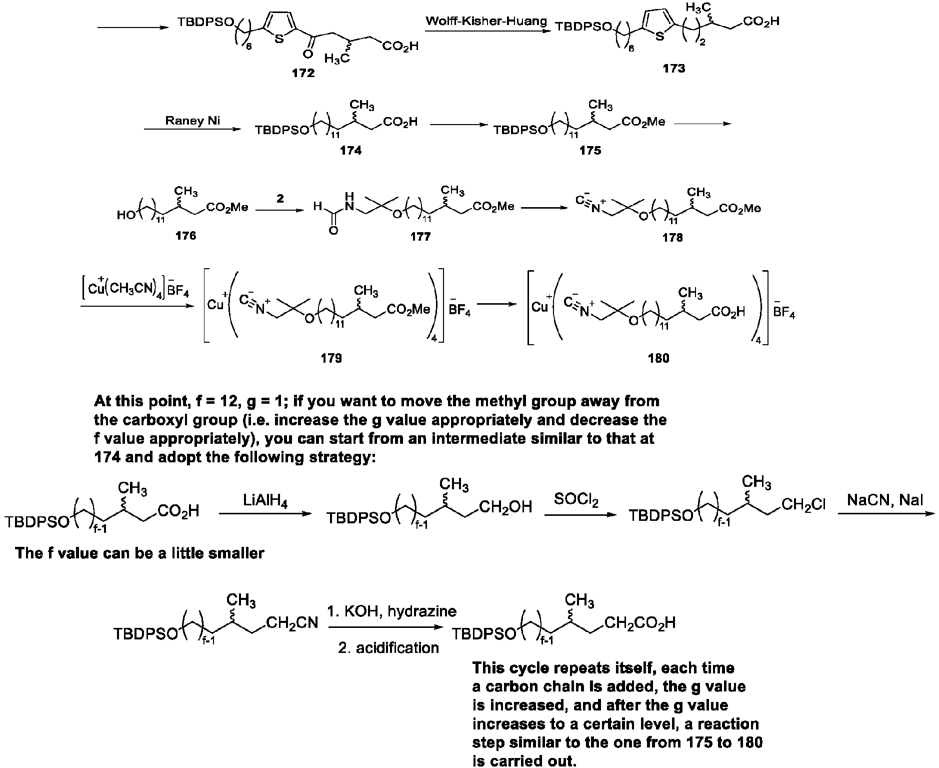
(2) The initial synthesis steps are as follows:
   First, 6-caprolactone **(164)** was ring-opened with sodium hydroxide and then acidified to give intermediate **165;**
   Then, intermediate **165** reacts with tert-butyldiphenylsilyl chloride (TBDPSCl) in DMF (imidazole is used as a base to participate in the reaction), and the alcoholic hydroxyl group of intermediate **165** is protected by *tert-*butyldiphenylsilyl chloride to convert into intermediate **166;**
   Next, intermediate **166** reacted with oxalyl chloride in toluene, and the carboxyl group of intermediate **166** was converted into an acid chloride to obtain intermediate **167;**
   Next, intermediate **167** reacted with thiophene **(168)** in dichloromethane to undergo Friedel-Crafts reaction (tin tetrachloride participated in the reaction as Lewis acid) to give intermediate **169;**
   Next, intermediate **169** reacted with potassium hydroxide and hydrazine in ethylene glycol, and underwent Wolff-Kishner-Huang Minlon Reduction, whereby the carbonyl group on intermediate **169** was reduced to a methylene group to obtain intermediate **170;**
   Subsequently, intermediate **170** reacted with 3-methylglutaric anhydride **(171)** in nitrobenzene to underwent a Friedel-Crafts reaction (aluminum trichloride participated in the reaction as a Lewis acid) to obtain intermediate **172;**
   Next, intermediate **172** was subjected to Wolff-Kishner-Huang Minlon Reduction, and the carbonyl group on intermediate **172** was reduced to methylene to obtain intermediate **173;**
   Then, the thiophene ring on intermediate **173** was reduced to four methylene groups -(CH₂)₄- by the action of Raney Ni to obtain intermediate **174;**
   Next, intermediate **174** was methylated in methanol using p-toluenesulfonic acid to obtain intermediate 175.
   Subsequently, the intermediate **175** was treated with tetrabutylammonium fluoride (TBAF) in a mixed solvent of tetrahydrofuran and acetic acid to undergo a deprotection reaction of the *tert*-butyldiphenylsilyl chloride protecting group to obtain the intermediate **176;**
   Then, referring to the specific operation steps in "Synthesis of Intermediate **29"** in Example 1 of the present invention, intermediate **176** and intermediate 2 were subjected to alkoxymercuration-demercuration reaction under the action of mercuric acetate and then sodium borohydride to obtain intermediate **177;**
   Next, referring to the specific operation steps in "Synthesis of isocyanomethyl ester intermediate **39"** in Example 1 of the present invention, intermediate **177** was dehydrated under the action of triethylamine and phosphorus oxychloride to obtain isocyanomethyl ester intermediate **178;**
   Subsequently, the isocyanomethyl ester intermediate **178** underwent a ligand exchange reaction with tetrakis(acetonitrile)copper(I) tetrafluoroborate to afford the copper salt methyl ester intermediate **179;**
   Next, the copper salt methyl ester intermediate **179** was hydrolyzed and acidified to obtain the copper salt carboxylic acid labeled precursor **180;**
   After that, the precursor **180** was labeled with copper salt carboxylic acid, and the rhenium complex and ^{99m}Tc complex thereof could be obtained by referring to the corresponding steps of "labeling of Re complex **69"** and "labeling of ^{99m}Tc complex **69"** in the above embodiment 1. No further description will be given here.

In addition, at this time, f = 12, g = 1; if you want to move the methyl group away from the carboxyl group (i.e., appropriately increase the g value in general formula VII and appropriately reduce the f value in general formula VII), you can start from an intermediate similar to **174** and adopt the following strategy: first, the carboxylic acid is reduced to a primary alcohol by lithium aluminum hydride, and the primary alcohol reacts with dichloride to become a chloride; the chloride reacts with sodium cyanide and sodium iodide to become a cyano group, and then hydrolyzes to a carboxylic acid, thereby increasing the g value in general formula VII, and obtaining a product in which the methyl substituent is away from the carboxyl group.

### Example 13

In addition, the synthetic route and method of the labeled precursor **190** of the typical compound of general formula **VIII** in this application are selected (in this embodiment, when f is 12 and g is 1 in general formula **VIII).** In addition, after the synthetic route of the labeled precursor 190, there is also a method for increasing the g value when f is small, as shown below:
(1) The synthetic route is as follows: 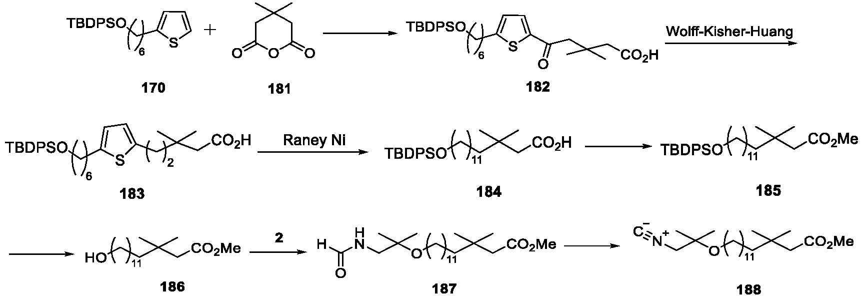 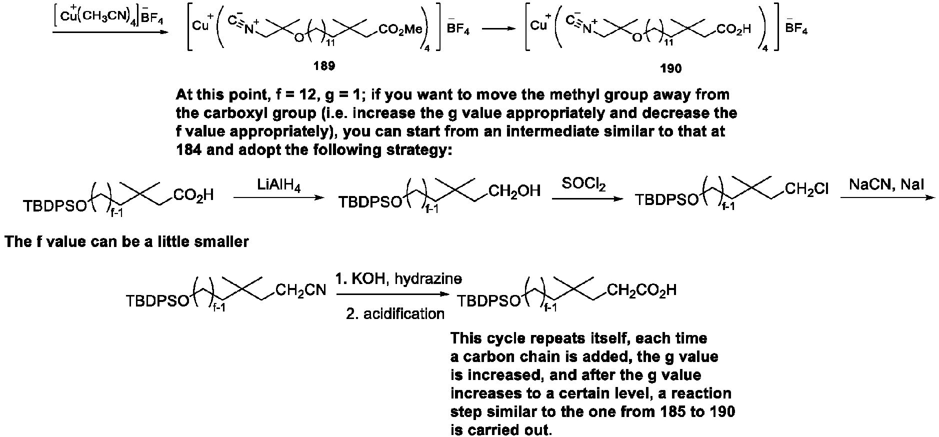
(2) The preliminary synthesis steps are as follows: The reaction steps from the intermediate **170** to the copper salt carboxylic acid labeling precursor **190** were completely similar to the reaction steps from the intermediate **170** to the copper salt carboxylic acid labeling precursor **180** in the above Example 13, and no further description was needed here.

After that, the precursor **190** is labeled with copper salt carboxylic acid, and the rhenium complex and ^{99m}Tc complex thereof can be obtained by referring to the corresponding steps of "labeling of Re complex **69"** and "labeling of ^{99m}Tc complex **69"** in the above embodiment 1. No further details will be given here.

In addition, at this time, f = 12, g = 1; if one wants to move the methyl group away from the carboxyl group *(i.e.,* appropriately increase the g value in the general formula **VIII** and appropriately reduce the f value in the general formula **VIII),** one can start from an intermediate similar to **184** and take reaction steps that are completely similar to those in the above Example 13 starting from the intermediate **174** to increase the g value, thereby increasing the g value in the general formula VII and obtaining a product in which the methyl substituent is away from the carboxyl group.

In the present invention, "calcd" refers to the predicted value of the molecular ion peak of the target molecule using Chemdraw software, "found" refers to the measured value of the molecular ion peak of the target molecule using a mass spectrometer; "HRMS" refers to high-resolution mass spectrometry characterization; the letter "M" in the HRMS characterization data of the copper methyl ester intermediate and the copper carboxylic acid labeled precursor refers to the Exact Mass (precise molecular ion peak) of the corresponding isocyanate monomer. The copper methyl ester intermediate and the copper carboxylic acid labeled precursor in the present invention can only display the molecular ion peak of "M+Cu" (corresponding isocyanate monomer plus copper atom) or "2M+Cu" (2 corresponding isocyanate monomers plus copper atom) in the positive ion characterization mode of the mass spectrometer.

It should also be noted that the present application used mice and rats for effect tests, because rat tests are closer to human test results, so rats have higher accuracy and greater reference significance, so rat tests were added on the basis of mice. In addition, due to space constraints, the structural characterization in this application only records specific characterization data, and omits the drawings.

The above description is only a preferred embodiment of the present invention and is not intended to limit the present invention. For those skilled in the art, the present invention may have various modifications and variations. Any modification, equivalent replacement, improvement, etc. made within the spirit and principle of the present invention shall be included in the protection scope of the present invention.

## Claims

1. A labeled fatty acid derivative, the general formula of which is shown in Formula I: 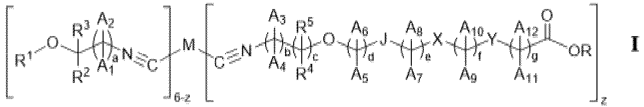 Wherein,
M is ^{99m}Tc or Re;
R, R¹, R², R³, R⁴ and R⁵ are independently H, aliphatic chain or alicyclic; A₁~A₁₂ are independently H, aliphatic chain or alicyclic;
J, X, Y are independently none, -O-, -S-, 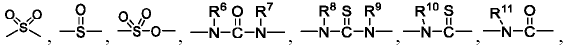 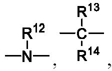 and J, X, Y cannot be none at the same time, R⁶~R¹⁴ are independently H or aliphatic chain, and R¹³~R¹⁴ cannot be H at the same time; z is an integer from 1 to 6, a is an integer from 0 to 5, b is an integer from 0 to 5, c is 0 to 3, and preferably b and c are not 0 at the same time; d is an integer from 0 to 27, e is an integer from 0 to 27, f is an integer from 0 to 28, and g is an integer from 0 to 7, and at least two of d, e, f and g are not 0.

2. The labeled fatty acid derivative according to claim 1, wherein at least one of J, X, and Y is an etheroxy group or a group containing a S atom, or the group 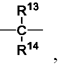 the group containing a S atom includes at least one of -S-,

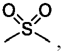

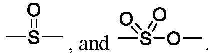

3. The labeled fatty acid derivative according to claim 2, wherein, J, X, and Y are independently none, -O-, -S-, 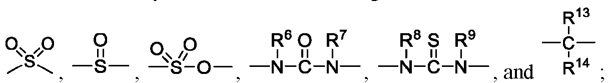 preferably, J is none, 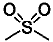 (sulfone), 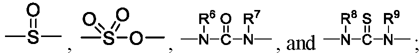 X is none, 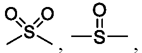 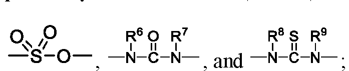 Y is -O-, -S-, 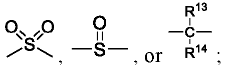 more preferably, at least one of J, X, and Y is a group containing S atoms or 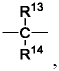 and the group containing S atoms includes one of -S-,

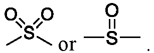

4. The labeled fatty acid derivative according to claim 1, wherein, in the general formula I, J, X, and Y are any one of the following combinations:
J and X are both none, and Y is -S-, 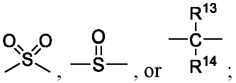
J is none, X is 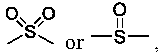
and Y is -S- or 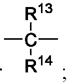
J and X are both 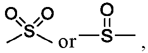
and Y is -S- or 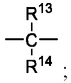
J is none, X is 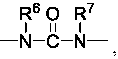
and Y is -S- or

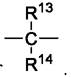

5. The labeled fatty acid derivative according to claim 1, wherein, R, R¹, R², R³, R⁴ and R⁵ are all H or an aliphatic chain; preferably, the aliphatic chain comprises an aliphatic hydrocarbon; more preferably, the aliphatic chain is an aliphatic hydrocarbon with 1 to 28 carbon atoms.

6. The labeled fatty acid derivative according to claim 1, wherein, a is an integer of 1-3; b is an integer of 1-3; c is an integer of 1-3; R, R¹~R¹⁴, and A₁~A₁₂ are independently -H or an aliphatic hydrocarbon of 1-5 carbon atoms, and R¹³~R¹⁴ cannot be H at the same time; d is an integer of 0-14, e is an integer of 0-14, f is an integer of 1-15, and g is an integer of 1-6.

7. The labeled fatty acid derivative according to claim 1, wherein, the structural formula of a labeled precursor of the fatty acid compound on the right side of general formula I is an isocyanate monomer 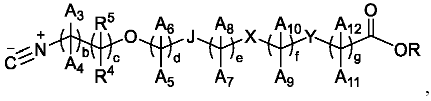 wherein J, X, Y, R⁴, R⁵, R, A₃~A₁₂, b, c, d, e, f and g are consistent with those defined in any one of claims 1-6; then the isocyanate monomer can be directly synthesized into general formula I by a one-step labeling method.

8. The labeled fatty acid derivative according to claim 1, wherein, the structural formula of a labeled precursor of the fatty acid compound on the right side of general formula I is an isocyanate metal salt 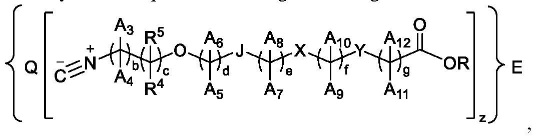 wherein Q is a metal cation, and the metal cation is preferably a copper ion, a cuprous ion, a calcium ion, a potassium ion, a sodium ion, a magnesium ion, an aluminum ion, and more preferably a copper ion or a cuprous ion; E is an anion, and the anion is preferably a tetrafluoroborate ion (BF₄⁻), a hexafluorophosphate ion (PF₆⁻), a trifluoroacetate ion (CF₃COO⁻), a perchlorate ion (ClO₄⁻), a fluoride ion, a chloride ion, a bromide ion, an iodide ion, and more preferably a tetrafluoroborate ion (BF₄⁻); J, X, Y, R⁴, R⁵, R, A₃~A₁₂, z, b, c, d, e, f and g are all consistent with those defined in any one of claims 1-6; then the isocyanate metal salt can be directly synthesized into general formula I by a one-step labeling method.

9. The labeled fatty acid derivative according to claim 1, wherein, M is ^{99m}Tc or Re, z is an integer of 1-6, a and b are both 1, c is 1, R, R¹, R², R³, R⁴ and R⁵ are independently -H, -CH₃ or -CH₂CH₃, A₁-A₁₂ are all H, then the general formula I includes the following compounds:
①When d and e are both 0, J and X are both none, f is an integer of 1-15, g is an integer of 1-6, and Y is -S-, the formula I is the following general formula **II:** 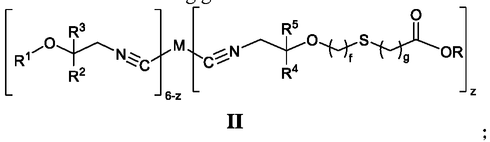
②When d and e are both 0, J and X are both none, f is an integer of 1-15, g is an integer of 1-6, and Y is the formula I is the following general formula **III:** 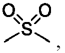 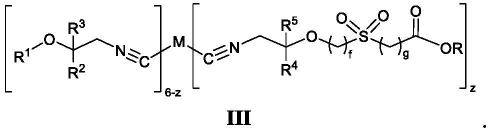
③When d is 0, J is none, e is an integer of 1-14, f is an integer of 1-15, g is an integer of 1-6, X is is -S-, the formula **I** is the following general formula **IV:** 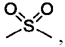 and Y 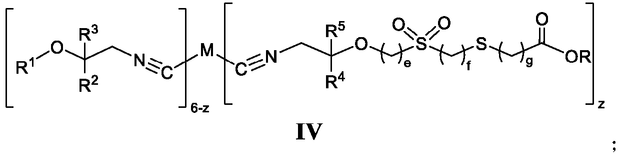
④When d and e are both integers of 1-14, f is an integer of 1-15, g is an integer of 1-6, J is and Y is -S-, the formula I is the following general formula V: 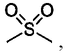 X is 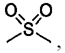 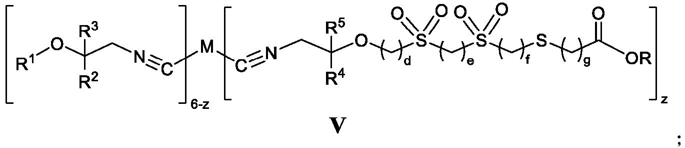
⑤When d is 0, J is none, e is an integer of 1-14, f is an integer of 1-15, g is an integer of 1-6, X is (urea bridge), and Y is -S-, the formula I is the following general formula VI: 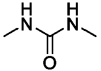 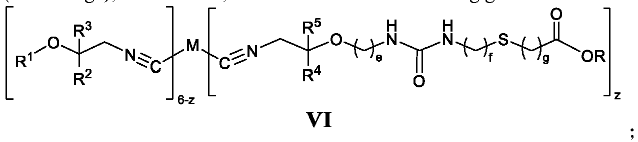
⑥When d and e are both 0, J and X are both none, f is an integer of 1-15, g is an integer of 1-6, and Y is 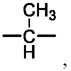 the formula I is the following general formula **VII:** 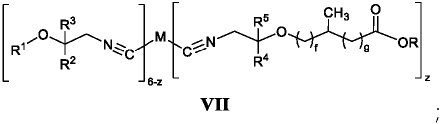
⑦When d and e are both 0, J and X are both none, f is an integer of 1-15, g is an integer of 1-6, and Y is the formula I is the following general formula **VIII:**

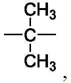

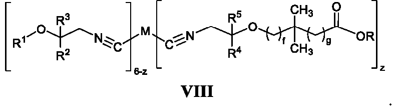

10. A precursor compound for preparing labeled fatty acid derivatives, wherein the precursor compound is an isocyanate monomer 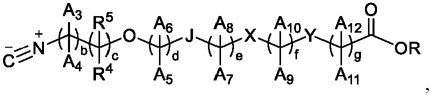 wherein J, X, Y, R⁴, R⁵, R, A₃~A₁₂, z, b, c, d, e, f and g are consistent with those defined in any one of claims 1-6.

11. A precursor compound for preparing labeled fatty acid derivatives, wherein the precursor compound is an isocyanate metal salt 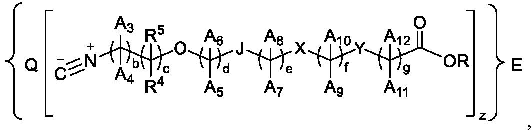 wherein Q is a metal cation, and the metal cation is preferably a copper ion, a cuprous ion, a calcium ion, a potassium ion, a sodium ion, a magnesium ion, an aluminum ion, and more preferably a copper ion or a cuprous ion; E is an anion, and the anion is preferably a tetrafluoroborate ion (BF₄⁻), a hexafluorophosphate ion (PF₆⁻), a trifluoroacetate ion (CF₃COO⁻), a perchlorate ion (ClO₄⁻), a fluoride ion, a chloride ion, a bromide ion, an iodide ion, and more preferably a tetrafluoroborate ion (BF₄⁻); J, X, Y, R⁴, R⁵, A₃~A₁₂, z, b, c, d, e, f and g are consistent with those defined in any one of claims 1-6.

12. A myocardial imaging agent, wherein the myocardial imaging agent includes the general formula I in any one of the above claims.
